# EUROPEAN PATENT SPECIFICATION

(11) **EP 1 577 673 B1**
(45) Date of publication and mention of the grant of the patent: **30.07.2008**
(21) Application number: 05005356.0
(22) Date of filing: 11.03.2005
(51) Int. Cl.: G01N 33/68

(54) **The use of BNP-type peptides and ANP-type peptides for assessing the risk of suffering from a cardiovascular complication as a consequence of volume overload**
Verwendung des BNP-Art und ANP-Art Peptiden für das Festsetzen der Gefahren von Herzgefäß- Komplikationen als Folge der Volumenüberlastung
L'utilisation des peptides de type BNP et ANP pour évaluer les risques des complications cardiovasculaires dues à une surcharge de volume

(30) Priority: 15.03.2004 EP 04006080
(43) Date of publication of application: 21.09.2005
(73) Proprietor: F. Hoffmann-La Roche AG, 4070 Basel (CH); Roche Diagnostics GmbH, 68305 Mannheim (DE)
(72) Inventor: Hess, Georg, 55130 Mainz (DE); Horsch, Andrea, 68259 Mannheim (DE)
(74) Representative: Isenbruck, Günter

(56) References cited:
- WO-A-01/14885
- US-A1- 2003 022 235
- RUSKOAHO H: "Cardiac hormones as diagnostic tools in heart failure" ENDOCRINE REVIEWS, BALTIMORE, MD, US, vol. 24, no. 3, 1 June 2003 (2003-06-01), pages 341-356, XP002303782
- REDFIELD MARGARET M ET AL: "Plasma brain natriuretic peptide concentration: Impact of age and gender" JOURNAL OF THE AMERICAN COLLEGE OF CARDIOLOGY, vol. 40, no. 5, 4 September 2002 (2002-09-04), pages 976-982, XP002335579 ISSN: 0735-1097
- AMBROSE T M: "REVIEW OF THE CLINICAL UTILITY OF NT-PROBNP IN THE DIAGNOSIS, PROGNOSIS, AND THERAPY MONITORING OF PATIENTS WITH CONGESTIVE HEART FAILURE" JOURNAL OF CLINICAL LIGAND ASSAY, CLINICAL LIGAND ASSAY SOCIETY, WAYNE, MI, US, vol. 25, no. 2, 21 June 2002 (2002-06-21), pages 160-166, XP008049569 ISSN: 1081-1672
- ANGERMANN CHRISTIANE E ET AL: "Natriuretic peptides - New diagnostic markers in heart disease" HERZ, vol. 29, no. 6, September 2004 (2004-09), pages 609-617, XP002335510 ISSN: 0340-9937

## Description

The present invention relates to the use of cardiac hormones for assessing the risk of suffering from a cardiovascular complication as a consequence of intravasal volume overload.

An aim of modem medicine is to provide personalized or individualized treatment regimens. Those are treatment regimens which take into account a patient's individual needs or risks. A particularly important risk is the presence of a cardiovascular complication, particularly an unrecognized cardiovascular complication.

Cardiovascular complications, particularly heart diseases, are the leading cause of morbidity and mortality in the Western hemisphere. Cardiovascular complications can remain asymptomatic for long periods of time. Therefore, reliable diagnosis of the presence of a cardiovascular complication is more difficult and error-prone than generally believed (Svendstrup Nielsen, L., et al. (2003). N-terminal pro-brain natriuretic peptide for discriminating between cardiac and non-cardiac dyspnoea. The European Jounal of Heart Failure)

It has been noted recently, that a small increase in intravasal volume (volume overload) can lead to a cardiovascular complication, possibly followed by cardiac decompensation and even death. Many pharmaceutical drugs cause fluid retention, either as wanted effects or unwanted side-effects. This can lead to intravasal volume increase, which in turn can lead to a cardiovascular complication or to deterioration of a pre-existing cardiovascular complication. For example, a diabetes drug, pioglitazone, has caused heart failure and build-up of fluid in lungs in 6 men with poor kidney or poor heart function (Reuters Health E-line 09/09/2003).

It has also been reported that transfusion of a single unit of erythrocytes (red blood cells) was sufficient to precipitate acute respiratory stress (dyspnea) in patients with an underlying but unrecognized cardiac or pulmonary disease. Similarly, platelet or plasma transfusions have been reported to cause volume overload (Kleinman, S., Chan, P., et al. (2003). Risks associated with transfusion of cellular blood components in Canada. Transfusion Medicine Reviews 17(2): 120-162).

Currently, only patients with a known history of heart disease or hypertension receive a closer monitoring, in case of a treatment resulting in an increase in intravasal volume. In particular, general practitioners and non-cardiologists have no means to identify a previously unrecognized cardiovascular problem.

In the prior art, no hint is given how the risk of a cardiovascular complication associated with volume overload can be diagnosed. Particularly, no reference has been made how such diagnosis can be made in patients that have no known history of cardiovascular complications.

Therefore, there is a need to for a method or means to identify risk patients before they receive treatment that results in volume overload. Particularly, there is a need to provide a suitable diagnostic means. Particularly, there is a need for a diagnostic means that allows to identify risk patients that have no history of a cardiovascular complication. In particular, the diagnostic means should be reliable and suited for use by general practitioners and non-cardiologists.

The object of the invention is attained by a method for diagnosing the risk of a patient who shows no symptoms of a cardiovascular disease according to the NYHA classification and who has no history of cardiovascular complications of suffering from a cardiovascular complication as a consequence of an increase of intravasal volume, comprising the steps of
a) measuring, preferably in vitro, the patient's level of a natriuretic peptide, from the group of ANP, NT-pro ANP and/or BNP, NT-proBNP
b) diagnosing the risk of the patient by comparing the measured level to at least one known level(s) associated with different grades of risk in a patient.

The object of the invention is also attained by use of a diagnostic means for measuring, preferably in vitro, a patient's level of a natriuretic peptide from the group of ANP, NT-proANP and/or BNP, NT-proBNP, for diagnosing the risk of a patient who shows no symptoms of a cardiovascular disease according to the NYHA classification and who has no history of cardiovascular complications, of suffering from a cardiovascular complication as a consequence of an increase of intravasal volume. Preferably the level is determined in a body fluid or tissue sample fo the patient.

The present invention provides simple and inexpensive methods and means to screen patients, who are presenting with volume overload or are about to receive medication or treatment resulting in volume overload, for their risk to develop a cardiovascular complication as a consequence of said volume overload. The present invention also provides levels of cardiac hormones indicating the existence or severity of a cardiovascular complication in patients with or without obvious symptoms of a cardiovascular complication.

Thes use of natriuretic peptides as molecular or biochemical markers is known as such. In WO 02/089657, it has been suggested to measure brain natriuretic peptide (BNP) to diagnose myocardial infarction. In WO 02/083913 it has been suggested to use BNP to predict near-term morbidity or mortality in patients with non-ST-elevated acute coronary syndromes.

US 2003/022235 uses cardiac hormones such as BNP and ANP as the preferred marker for assessing the cardiovascular risk. D1 discloses as well the prognostic markers related to BNP, including NT-proBNP, the pro domain, a fragment of the pre pro-BNP other than BNP and a fragment of the pro domain. However, US 2003/022235 is silent about patients showing no symptoms of a pre-existing cardiovascular diseases.

RUSKOAHO H: ENDOCRINE REVIEWS, (2003-06-01), teaches that measuring the level of natriuretic peptides such as, N-ANP, BNP and N-BNP, is a method of identifying (diagnosing) the risk of a patient for future cardiovascular events (complications). Moreover, the patients to whom the review relates are the symptomatic and asymptomatic patients with left ventricular dysfunction after myocardial infarction. The natriuretic peptide identified by RUSKOAHO H. as a predictor of a cardiovascular event (e.g. heart failure) in asymptomatic patients with left ventricular dysfunction after myocardial infarction is ANP.

The present invention is particularly advantageous to general practitioners, specialized physicians, and specialized wards, departments, or clinics which frequently have no access to extensive cardiological examination by cardiologists. The present invention provides means and methods to such non-cardiologists for simple and reliable screening of patients for those patients who are posed at risk of suffering from a cardiovascular complication as a consequence of an increase of intravasal volume.

The invention takes advantage of certain biochemical or molecular markers. The terms "biochemical marker" and "molecular marker" are known to the person skilled in the art. In particular, biochemical or molecular markers are gene expression products which are differentially expressed (i.e. upregulated or downregulated) in presence or absence of a certain condition, disease, or complication. Usually, a molecular marker is defined as a nucleic acid (such as an mRNA), whereas a biochemical marker is a protein or peptide. The level of a suitable biochemical or molecular marker can indicate the presence or absence of the condition, disease, or complication, and thus allow diagnosis.

The present invention particularly takes advantage of natriuretic peptides as biochemical markers. Also taking advantage of combinations of any natriuretic peptides as biochemical markers is considered in the context of the present invention.

Natriuretic peptides according to the present invention are ANP-type and BNP-type peptides (see e.g. Bonow, R.O. (1996). New insights into the cardiac natriuretic peptides. Circulation 93: 1946-1950).

ANP-type peptides comprise pre-proANP, proANP, NT-proANP, and ANP.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller T, Gegenhuber A, et al., Clin Chem Lab Med 2004; 42: 942-4, supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73.).

Preferred natriuretic peptides according to the present invention are NT-proANP, ANP, NT-proBNP and BNP. ANP and BNP are the active hormones and have a shorter half-life than their respective inactive counterparts, NT-proANP and NT-proBNP. Therefore, depending on the time-course that is of interest, either measurement of the active or the inactive forms can be advantageous. The most preferred natriuretic peptides according to the present invention are NT-proBNP.

The term "variants" in this context relates to peptides substantially similar to said peptides. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence similarity to the most prevalent isoform of the peptide of at least 80%, preferably at least 85%, more preferably at least 90%, most preferably at least 95%. Substantially similar are also proteolytic degradation products which are still recognized by the diagnostic means or by ligands directed against the respective full-length peptide.

The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide.

Other embodiments of the invention include the measuring of different cardiac hormones in combination, simutaneously or non-simultaneously. For example, measuring different cardiac hormones can yield important additional information, e.g. on the time course of an intravasal volume increase. For example, the level of NT-proBNP rises more slowly than the level of NT-proANP. On the other hand, after a volume increase, the level of NT-proBNP remains elevated for a longer period of time than the level of NT-proANP (see Example 2). Therefore, the present invention also relates to measuring both an ANP-type peptide and a BNP-type peptide. The present invention also relates to measuring the level of NT-proBNP at least 6 hours after onset of the intravasal volume increase. The present invention also relates to measuring the level of NT-proANP between 2 and 5 hours after onset of the intravasal volume increase.

Diagnosing according to the present invention includes determining,monitoring, confirmation, subclassification and prediction of the relevant risk, disease, or complication. Determining relates to becoming aware of a risk, disease, or complication. Monitoring relates to keeping track of an already diagnosed risk, disease, or complication, e.g. to analyze the progression of the risk, disease, or complication, or the influence of a particular treatment on the progression of the risk, disease, or complication. Confirmation relates to the strengthening or substantiating a diagnosis already performed using other indicators or markers.
Subclassification relates to further defining a diagnosis according to different subclasses of the diagnosed risk, disease, or complication, e.g. defining according to mild and severe forms of the risk, disease, or complication. Prediction relates to prognosing a risk, disease, or complication before other symptoms or markers have become evident or have become significantly altered.

Individuals suffering from a cardiovascular diseases can be individuals suffering from stable angina pectoris (SAP) and individuals with acute coronary syndromes (ACS). ACS patients can show unstable angina pectoris (UAP) or these individuals have already suffered from a myocardial infarction (MI). MI can be an ST-elevated MI or a non-ST-elevated MI. The occurring of an MI can be followed by a left ventricular dysfunction (LVD). Finally, LVD patients undergo congestive heart failure (CHF) with a mortality rate of roughly 15 %.

Cardiovascular diseases have been classified into a functional classification system according to the New York Heart Association (NYHA). Patients of Class I have no obvious symptoms of cardiovascular disease. Physical activity is not limited, and ordinary physical activity does not cause undue fatigue, palpitation, or dyspnea (shortness of breath). Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased.

Accordingly, patients can be divided into individuals showing no clinical symptoms and those with symptoms (e.g. dyspnea).

Another characteristic of cardiovascular diseases can be the "left ventricular ejection fraction" (LVEF) which is also known as "ejection fraction". People with a healthy heart usually have an unimpaired LVEF, which is generally described as above 50 %. Most people with a systolic heart disease which is symptomatic generally have an LVEF of 40 % or less.

The present invention relates to "cardiovascular complications" developing as a consequence of intravasal volume increase.

A "cardiovascular complication" according to the present invention relates to any cardivascular disease, event, or any secondary complication, e.g. pulmonary congestion or congested lung (which can result e.g. from left ventricular insufficiency).

Particularly, "cardiovascular complication" relates to coronary heart disease, SAP, ACS, UAP, MI, ST-elevated MI, non-ST-elevated MI, LVD, CHF, and pulmonary congestion.

More particularly, "cardiovascular complication" relates to ACS, UAP, MI, ST-elevated MI, non-ST-elevated MI, LVD, CHF, and pulmonary congestion.

A cardiovascular complication according to the present invention may cause symptoms, particularly symptoms according to NYHA class II-IV, more particularly according to NYHA class III-IV.

A cardiovascular complication may be associated with an LVEF of 40% or less.

A cardiovascular complication may either be "compensated" or "decompensated". Compensated means that the regular oxygen need of the body can still be satisfied, whereas decompensated means that the regular oxygen need of the body is not satisfied anymore.

"Suffering from a cardiovascular complication" according to the present invention also includes deterioration of a pre-existing cardiovascular complication.

The term "patient" according to the present invention relates to a healthy individual, an apparently healthy individual, or particularly an individual suffering from a disease. Particularly, the patient is suffering from or treated for diabetes, (diabetes type I or type II), rheumatism, rheumatoid arthritis, inflammatory diseases, or cancer. Even more particularly, the patient has no known history of cardiovascular complication, and no (NYHA class I or II) symptoms of a cardiovascular complication, and he is not being treated for a cardiovascular complication. However, also healthy volunteers who have no signs or history of a cardiovascular complication are considered to be patients according to the present invention.

Preferably, the patient is a patient whose intravasal volume is increased or will be increased. The intravasal volume increase may be present or it may be going to take place in the future.

Intravasal volume relates to the total volume of the cellular (e.g. erythrocytes) and non-cellular (blood plasma) blood components. The intravasal volume of an adult individual is typically in the range from 4 to 6 liters.

According to the present invention, a intravasal volume increase relates to an increase in intravasal volume of at least 5 %, particularly at least 10 % and more particularly at least 20 % of the intravasal volume of the patient. For example, an increase of a single unit of blood (500 ml, which roughly equals a 10% increase of intravasal volume), particularly at least two units, more particularly at least 3 units, is considered to be a intravasal volume increase according to the present invention.

A "transient" intravasal volume increase is an intravasal volume increase present only once within a given period of time. It is characterized by an increase and subsequent decrease in intravasal volume to a near-normal value within a short time period, particularly within 12 hours, more particularly within 6 hours, and most particularly within 30 minutes after onset of the intravasal volume increase.

An intravasal volume increase is considered to be "sustained" if it manifests itself more slowly than a "transient" intravasal volume increase and/or if it is present over a longer period of time, e.g. one day, several days, or weeks.

Examples for typical transient intravasal volume increases include oral application of liquids, and infusions or transfusions. E.g. drinking of water, soup, infusion of plasma, parenteral administration of nutrients and blood transfusions typically cause a transient increase of intravasal volume. "Infusions" include, but are not limited to parenteral or intravenous infusions of blood, plasma, erythrocytes, thrombocytes, electrolytes, antibiotics or other medicaments, or nutrients. "Transfusions" particularly include transfusions of blood, plasma, erythrocytes, thrombocytes, or electrolytes.

Examples for a sustained increase include the constant intravenous application of liquids, and particularly the administration of drugs that cause water retention.

Even a small but sustained increase in intravasal volume can put considerable strain on the cardiovascular system. Therefore, a sustained increase can be particularly dangerous to the patient. A sustained increase may be terminated or treated for example by application of diuretics. Further examples for treatment options are given below.

It is known to the person skilled in the art, under what circumstances a cardiovascular complication can be considered to occur "as a consequence" of the intravasal volume increase. Particularly, a cardiovascular complication is considered to occur as a consequence of the intravasal volume increase, if it occurs within one day, particularly within 12 hours, more particularly within 4 hours after onset of a transient intravasal volume increase. Alternatively, a cardiovascular complication is considered to occur as a consequence of the intravasal volume increase, if occurs within a day, a few days or a few weeks after onset of a sustained intravasal volume increase.

The intravasal volume increase may be caused by disease or artificially. Examples for diseases causing intravasal volume increase include sepsis and diseases which cause an increase of intravasal protein concentrations (e.g. gammopathies).

Sepsis, or blood poisoning, can lead to serious disturbance of the water balance. Decreases of intravasal volume can be followed by increases of intravasal volume and vice versa, frequently in rapid succession. Additionally, treatment may require the infusion of large amounts of liquid (up to several liters). The present invention allows to closely monitor any risks of a cardiovascular complication to develop as a consequence of increases in intravasal volume. Thus, treatment of the primary disease can be adapted to this risk, or it can be accompanied by cardiovascular medication. The effect of a substitution of lost intravasal volume or an administration of vasopressive drugs can be closely monitored by measuring the level of a cardiac hormone according to the present invention.

Artificial intravasal volume increase may be due to medical treatment or experimental treatment.

Medical treatment leading to intravasal volume increase includes oral administration of liquids, infusions, transfusions, and administration of drugs which cause water retention.

Intravasal volume increase caused by administration of drugs usually takes longer to develop than an increase caused by infusions, transfusions, or orally administered liquids. Therefore, drugs typically induce a slow but sustained volume increase, whereas infusions, transfusions, or orally administered liquids cause a rapid but transient volume increase.

Drugs causing water retention are known to the person skilled in the art. Particularly, such drugs include anti-inflammatory drugs (including non-steroid anti-rheumatics, Cox-2 inhibitors, particularly selective Cox-2 inhibitors, corticosteroids), diabetes drugs, estrogens, and TNF inhibitors.

Examples for anti-inflammatory drugs (including non-steroidal anti-rheumatics (also referred to as non-streoidal anti-inflammatory drugs) include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Cox-2 inhibitors (particularly specific or "selective" Cox-2 inhibitors, more particularly Celecoxib, Rofecoxib (VIOXX)); Deflazacort; Desonide; Desoximetasone; Dexamethasone Dipropionate; Diclofenac; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluormetholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminium; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Soidum; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium.

Examples for corticosteroids include cortisone; fluocortolone; hydrocortisone; methylprednisolone; prednisolone; prednisone; prednylidene.

Examples for diabetes drugs include thiazolidinedones, for example glitazone; medione; pioglitazone; rosiglitazone; troglitazone. Also combinations of such drugs with insulin, sulfonylurea, and metformin are diabetes drugs according to the present invention.

Estrogens can be natural or synthetic, conjugated or unconjugated. Examples for estrogens include estradiol; estriol; estradiolvalerate; estrone; ethinylestradiol; mestranol.

Examples for TNF inhibitors include Etanercept and Infliximab.

It has been noted recently, that selective Cox-2 inhibitors can lead to cardiovascular complications, possibly followed by cardiac decompensation and even death. In a recent study (APPROVE study (Adenomatous Polyp Prevention On Vioxx)) even in the early post-observation phase higher blood pressure levels had been noticed with 25 mg rofecoxib than with a placebo. Only patients without any recognisable cardiovascular risk were included in that study for the secondary prevention of colon adenomas.

The term "non-steroidal anti-rheumatics" (also referred to as non-steroidal anti-inflammatory drugs or NSAIDs) is known to the person skilled in the art. NSAIDs inhibit cyclooxygenases (also known as prostaglandin-H-synthetases). Cyclooxygenases catalyze the reaction from arachidonic acid to prostaglandin H₂ (a cyclic endoperoxide), which is the precursor of prostaglandin I₂ (also known as prostacycline), thromboxan A₂, and other prostaglandins. Prostaglandins play a significant role in pain, fever, and inflammatory reactions. There are two isoforms of cyclooxygenases, Cox-1 and Cox-2. The Cox-2 gene is an immediate early gene and is induced under conditions of tissue damage, pain reactions, or inflammatory reactions. Thus, NSAIDs include Cox-1 inhibitors and Cox-2 inhibitors. The NSAIDs may inhibit both isoforms or they may be selective for one isoform (i.e. they inhibit only one of the two isoforms at the therapeutic dosage).

Examples for unspecific NSAIDs include Ibuprofen; Flurbiprofen; Naproxen; Flufenamic Acid; Mefenamic Acid; Piroxicam; Diclofenac; Phenbutazone Sodium Glycerate; Indometacin; Tenoxicam.

Selective Cox-2 inhibitors according to the present invention are compunds which, under therapeutic conditions, do inhibit expression or, preferably, the enzymatic function of Cox-2, whereas not significantly inhibiting expression or, preferably, the enzymatic function of Cox-1.

Examples for selective Cox-2 inhibitors include coxibes (e.g. celecoxib, rofecoxib, etoricoxib, valdecoxib, parecoxib (a pro-drug of valdecoxib), lumiracoxib), meclofenatmate, sulindac sulphide, diclofenac, nimesulide, meloxicam, etodolac, NS398, L-745,337, DFP (3-(2-propyloxy)-4-(4-methylsulphonylphenyl)-5,5-dimethylfuranone). The latter three compounds are described in Warner, T.D., et al., 1999).

The enzymatic function of the two cyclooxygenases can be measured according to methods known in the art, including suitable in vivo or in vitro tests. A typical marker for the enzymatic function of Cox-1 is the formation of thromboxan A₂, whereas a typical marker for the enzymatic function of Cox-2 is the formation of prostaglandins (e.g. prostaglandin E₂ from macrophages.

Examples for a suitable test systems have been published (e.g. Warner, T.D., Giuliano, F., Vojnovic, I., et al. (1999). Nonsteroid drug selectivities for cyclo-oxygenase-1 rather than cyclo-oxygenase-2 are associated with human gastrointestinal toxicity: A full in vitro analysis. Proceedings of the National Academy of Sciences USA, vol. 96., pp. 7563-7568, a relevant erratum has been published in vol. 96(17), p. 9966d). This assay will be referred to as the William Harvey Modified Assay. The assay is described in detail in Warner T.D., et al. supra, on page 7563-4, the description of which is expressly incorporated herein by reference.

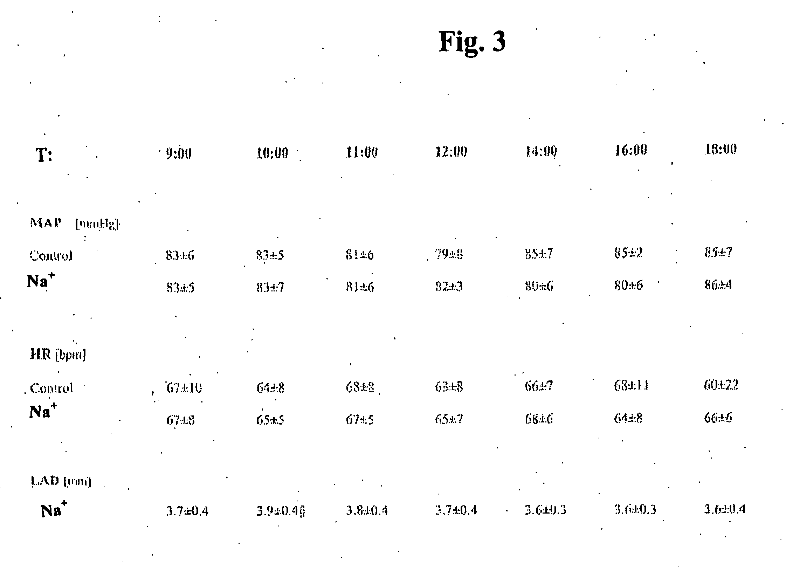

Preferably, a selective Cox-2 inhibitor according to the present invention is more than 5-fold Cox-2 selective according to the William Harvey Modified Assay, more preferably more than 50-fold Cox-2 selective according to the William Harvey Modified Assay (see Warner, T.D. et al., supra, Fig. 3 on page 7567).

Alternatively, the selective Cox-2 inhibitor according to the present invention is a compound preferably being more selective for Cox-2 than diclofenac, more preferably being more selective for Cox-2 than nimesulide, even more preferably at least as selective as for Cox-2 as celecoxib under therapeutic conditions.

In another preferred embodiment, the present invention relates to means and methods for diagnosing the risk of a patient who shows no symptoms of a cardiovascular disease according to the NYHA classification and who has no history of a cardiovascular complication of suffering from a cardiovascular complication as a consequence of the increase of intravasal volume, wherein the increase of intravasal volume is caused artificially, by infusion or transfusion of liquids, or by administration of a "coxibe". Examples for coxibes include celecoxib (Celebrex™, Pfizer), rofecoxib (VIOXX™, Merck), etoricoxib, valdecoxib, parecoxib (a pro-drug of valdecoxib), lumiracoxib (Prexige™, Novartis). Other similar compounds, several of which are under development and examination, are also included in the scope of the present invention.

Also experimental treatment can lead to intravasal volume increase. Notably, also tilting of the body can simulate increase of intravasal volume and lead to a release of cardiac hormones. Thus, the present invention also relates to a method for diagnosing the risk of a patient of suffering from a cardiovascular complication as a consequence of an increase of intravasal volume, said method comprising the additional step of tilting the patient before the cardiac hormone, preferably a natriuretic peptide, is measured. Tilting, combined with a method of diagnosis according to the present invention, allows to diagnose the risk in a carefully controlled medical environment. As the strain on the cardiovascular system by tilting is reversible, a tilting procedure can provide valuable diagnostic information in a safe experimental setting.

Tilting may also serve to assess a healthy volunteer's risk of suffering from a cardiovascular complication. In particular, the tilting procedure may be advantageous in physical examinations of persons experiencing sudden changes in blood pressure or blood distribution, such as pilots; skydivers, bungee jumpers, and astronauts.

According to the present invention, tilting relates to any means capable of redistributing blood to the upper body as compared to the blood distribution in the standing or supine position. Examples include tilting of the body head down, the use of gravitational or centrifugal force, and the use of pressure suits.

In particular, tilting relates to tilting the body of the patient, head down, by 5-90°, preferably 10-30°, more preferably 10-20°, most preferably 15°. As a control, the tilting protocol may include tilting the body of the patient feet down by the respective degrees of tilt.

Diagnosis according to the present invention is preferably done by use of a diagnostic means. A diagnostic means is any means that allows to measure the level amount, or concentration of a substance of interest, particularly a peptide or polypeptide of interest, more particularly a cardiac hormone.

In another embodiment, the present invention relates to a method of deciding about administering to a patient an infusion, a transfusion, or a drug causing volume overload, comprising (a) measuring, preferably in vitro, the level of a natriuretic peptide from the group of ANP, NT-proANP and/or BNP, NT-proBNP in the patient who shows no symptoms of a cardiovascular disease according to the NYHA classification and who has no history of cardiovascular complication, (b) comparing the measured level with at least one known level(s) associated with different grades of risk in a patient, (c) optionally initiating an examination of the patient by a cardiologist, (d) recommending or refraining from administering the infusion, transfusion, or drug, optionally in consideration of the result of the patient's examination by the cardiologist. It is evident that this method may be adapted according to all embodiments of the invention as mentioned earlier in this specification. Particularly, the method is for deciding about administering to a patient a drug causing volume overload and the drug being a selective Cox-2 inhibitor. Furthermore, the preferred cardiac hormone is a BNP-type peptide, particularly BNP or NT-proBNP.

Recommending or refraining from administering the infusion, transfusion, or drug is preferably based upon the risk indicated by comparing the measured level to the at least known level. As already laid out earlier, if the measured level indicates no increased risk, then treatment may be recommended. Recommending administration of the infusion, transfusion, or drug will preferably be done if other cardiovascular risk factors (e.g. the Framingham score, which is well-known to the cardiologist) also indicate a low risk of suffering from a cardiovascular complication. If the measured level indicates an increased risk, then administering may be recommended, but it is preferably accompanied (or "monitored") by further measuring of the level of the cardiac hormones of the invention and by further diagnosis, such as electrocardiography, echocardiography, or any other suitable methods known to the skilled cardiologist. If the measured level indicates a highly or very highly increased risk, then it is preferably refrained from administering the infusion, transfusion, or drug.

Optionally, the patient is examined by a cardiologist. This examination is preferably done if the measured level indicates an increased, highly increased, or very highly increased risk. The cardiologist may examine the patient according to any methods or means known and deemed appropriated. Recommending or refraining from administering the infusion, transfusion, or drug may be made in consideration of the risk as indicated according to the present invention and the result of an examination by a cardiologist.

Methods and diagnostic means which can be used to determine the levels of the respective peptides are known to the person skilled in the art. These methods include microplate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Furthermore, the person skilled in the art is familiar with different methods of measuring the level of a peptide or polypeptide. The term "level" relates to amount or concentration of a peptide or polypeptide in a patient or a sample taken from a patient.

The term "measuring" according to the present invention relates to determining the amount or concentration, preferably semi-quantitatively or quantitatively, of the nucleic acid, peptide, polypeptide, or other substance of interest. Measuring can be done directly or indirectly. Indirect measuring includes measuring of cellular responses, bound ligands, labels, or enzymatic reaction products.

In the context of the present invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In a preferred embodiment, the method for measuring the level of a peptide or polypeptide of interest, particularly a cardiac hormone, comprises the steps of (a) contacting a cell capable of a cellular response to the peptide or polypeptide with the peptide or polypeptide for an adequate period of time, (b) measuring the cellular response.

In another preferred embodiment, the method for measuring the level of a peptide or polypeptide of interest, particularly a cardiac hormone, comprises the steps of (a) contacting a peptide or polypeptide with a suitable substrate for an adequate period of time, (b) measuring the amount of product.

In another preferred embodiment, the method for measuring the level of a peptide or polypeptide of interest, particularly a cardiac hormone, comprises the steps of (a) contacting a peptide or polypeptide with a specifically binding ligand, (b) (optionally) removing non-bound ligand, (c) measuring the amount of bound ligand.

Preferably, the peptide or polypeptide is contained in a sample, particularly a body fluid or tissue sample, and the amount of the peptide or polypeptide in the sample is measured.

Peptides and polypeptides (proteins) can be measured in tissue, cell, and body fluid samples, i.e. preferably in vitro. Preferably, the peptide or polypeptide of interest is measured in a body fluid sample.

A tissue sample according to the present invention refers to any kind of tissue obtained from the dead or alive human or animal body. Tissue samples can be obtained by any method known to the person skilled in the art, for example by biopsy or curettage.

Body fluids according to the present invention may include blood, blood serum, blood plasma, lymphe, cerebral liquor, saliva, and urine. Particularly, body fluids include blood, blood serum, blood plasma, and urine. Samples of body fluids can be obtained by any method known in the art.

Methods to obtain cell samples include directly preparing single cells or small cell groups, dissociating tissue (e.g. using trypsin), and separating cells from body fluids, e.g. by filtration or centrifugation. Cells according to the present invention comprise also platelets and other non-nuclear cells, e.g. erythrocytes.

If necessary, the samples may be further processed. Particularly, nucleic acids, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a peptide, polypeptide, or a small molecule.

Other preferred methods for measurement may include measuring the amount of a ligand binding specifically to the peptide or polypeptide of interest. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from the human or animal body, can be modified, e.g. by glycosylation. A suitable ligand according to the present invention may bind the peptide or polypeptide also via such sites.

Preferably, the ligand should bind specifically to the peptide or polypeptide to be measured. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide.

Non-specific binding may be tolerable, particularly if the investigated peptide or polypeptide can still be distinguished and measured unequivocally, e.g. according to its size on a Western Blot, or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with an detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated.

Typical radioactive labels include ³⁵S, ¹²⁵I, ³²P, ³³P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)₂ fragments that are capable of binding antigen or hapten. The present invention also includes "humanized" hybrid antibodies wherein amino acid sequences of a non-human donor antibody exhibiting a desired antigen-specificity are combined with sequences of a human acceptor antibody.

The donor sequences will usually include at least the antigen-binding amino acid residues of the donor but may comprise other structurally and/or functionally relevant amino acid residues of the donor antibody as well. Such hybrids can be prepared by several methods well known in the art.

In another preferred embodiment, the ligand, preferably chosen from the group consisting of nucleic acids, peptides, polypeptides, more preferably from the group consisting of nucleic acids, antibodies, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

The method according to the present invention comprises the step of diagnosing the risk of the patient by comparing the measured level to known levels associated with different grades of risk in a patient.

The person skilled in the art is able to determine known levels of cardiac hormones which are associated with different grades of risk of suffering from a cardiovascular complication as a consequence of an increase of intravasal volume.

According to the present invention, the term "risk" relates to the probability of a particular incident, more particularly a cardiovascular complication, to take place. The grade of risk can be increased, highly increased, or very highly increased. The grade of risk can also not be increased. "No increased risk" means that there is apparently no risk of suffering from a cardiovascular complication as a consequence of an increase of intravasal volume.

Guidance as to what levels are associated with which grad of risk can be drawn from levels of cardiac hormones known to be associated with the presence or severity of a cardiovascular disease. For example, based on a 97.5 percentile obtained in individuals below the age of 50, a plasma level of 125 pg/ml of NT-proBNP was considered a normal level (see Example 3). Higher levels of NT-proBNP correlate for example with the level of symptoms according to the NYHA classification and with the level of impairment of LVEF. The term "plasma level" relates to levels of NT-proBNP measured in blood plasma.

Below, plasma levels of NT-proBNP are given which are typically considered to be associated with the indicated grades of risk of suffering from a cardiovascular complication as a consequence of an increase of intravasal volume.

It is evident, that the levels given below can serve only as a first classification of the risk of a patient. For example, the risk is also dependent on the spare pumping capacity of heart of the particular patient.

Furthermore, the person skilled in the art is able to determine other relevant levels from the Examples shown further below, particularly levels which are relevant in certain patient populations, such as elderly patients or patients with a increased or decreased levels of markers for thyroid function (e.g. TSH or FT4).

Typically, a plasma level of less than 50 pg/ml of NT-proBNP is associated with no increased risk of suffering from a cardiovascular complication as a consequence of an increase of intravasal volume. Particularly, in male patients a plasma level of less than approximately 60 to 100 pg/ml is associated with no increased risk, whereas in female patients a plasma level of less than approximately 120 to 150 pg/ml is associated with no increased risk. The average value is 125 pg/ml.

Typically, a plasma level higher than the plasma level for no increased risk but lower than 1000 pg/ml of NT-proBNP is associated with an increased risk of suffering from a cardiovascular complication as a consequence of an increase of intravasal volume.

Typically, a plasma level from 1000 to 5000 pg/ml of NT-proBNP is associated with a highly increased risk of suffering from a cardiovascular complication as a consequence of an increase of intravasal volume.

Typically, a plasma level of more than 5000 pg/ml of NT-proBNP is associated with a very highly increased risk of suffering from a cardiovascular complication as a consequence of an increase of intravasal volume.

Once the risk in a patient has been diagnosed, it may have consequences for the subsequent treatment as described below. The grades of risk mentioned below particularly refer to the grades of risk associated with the above described levels of NT-proBNP.

If a method according to the present invention indicates no increased risk, then treatment may be continued as planned.

If a method according to the present invention indicates an increased risk, then treatment may be adapted. Preferably, treatment will be accompanied by further measuring of the level of the cardiac hormones of the invention and by further diagnosis, such as electrocardiography, echocardiography, or any other suitable methods known to the skilled cardiologist.

If a method according to the present invention indicates a highly increased risk, then treatment may be adapted as described for increased risk. However, it may also be reconsidered if any intravasal volume increase can be tolerated, for example whether an artificial increase of intravasal volume shall be evoked at all.

If a method according to the present invention indicates a very highly increased risk, then treatment may be adapted as described for highly increased risk. However, also immediate hospitalization and/or intensive cardiac treatment may be considered.

Adapting treatment may include measures such as limitation of any intravasal volume increase present or planned, restriction of salt intake, regular moderate exercise, avoidance of non-steroidal anti-inflammatory agents, providing influenzal and pneumococcal immunization, administering drugs such as diuretics (including co-administration of more than one diuretic), ACE inhibitors, β-adrenergic blockers, angiotensin-receptor blockers, digitalis and any other measures known and deemed appropriate by the person skilled in the art. Therefore, the present invention also provides a method of treating a patient whose intravasal volume is increased or will be increased.

### Figure Legends

- Fig. 1: Hemodynamic data in 16 healthy volunteers undergoing tilting manoeuvres. Data are mean ± standard deviation. SABP, DABP, and MAP systolic , diastolic, and mean arterial blood pressure; HR: heart rate; LAD: left atrial diameter; Data for LAD are given in percent of baseline (BL). *: between group differences p <0.05; ANOVA repeated measures.

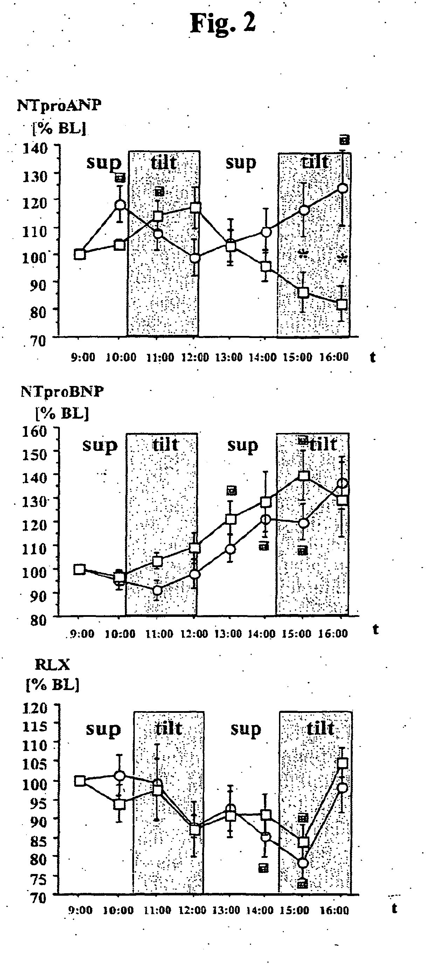

- Fig. 2: The couse of plasma levels of NT-proANP (upper panel), NT-proBNP (middle panel), and relaxin (RLX) in 16 healthy volunteers subjected to sequential tilting manoeuvres in two groups of n=8. Circles denote subjects positioned into the feet-down position first and in the head-down position afterwards; squares denote subjects subjected to an opposite tilting sequence. Data are given as percent of baseline values set to 100% as mean ± SEM. *: between group difference p <0.05; ANOVA repeated measures. 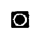 : significant (p < 0.05) difference in comparison with baseline values (paired student's t-test).
- Fig. 3: Hemodynamic data in 10 healthy volunteers in the control and the sodium loading (intravasal volume increase, Na⁺) group. Data are mean ± standard deviation (absolute data). MAP: mean arterial blood pressure; HR: heart rate; LAD: left atrial diameter; T: time; *: between group differences p <0.05; Friedman's test followed by Wilcoxon's matched pairs test. § intraindividual difference in comparison with baseline levels p >0.05.
- Fig. 4: The course of plasma levels of NT-proANP (upper panel), NT-proBNP (middle panel), and relaxin (RLX) in 10 healthy volunteers during the control-protocol (circles) and the sodium-loading (intravasal volume increase) protocol (squares). Data are given as mean ± SEM. *: between group difference p <0.05; 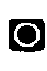 : significant (p < 0.05) difference in comparison with baseline values (Wilcoxon's matched pairs test).

- Fig. 5: The course of urine flow (UV), fractional excretion of sodium (FE_{Na}), and creatinine clearance (C_{Crea}) in 10 healthy volunteers during the sodium-loading (intravasal volume increase) protocol. Data are given as mean ± SEM. †: significant (p < 0.05) difference in comparison with baseline values. §: significant (p < 0.05) difference in comparison with 10:00 values (before sodium infusion); Wilcoxon's matched pairs test.
- Fig. 6: The course of urinary excretion of NT-proBNP (U_{NT-proBNP}), urinary excretion of relaxin (U_{RLX}), and urodilatin (U_{URO}) in 10 healthy volunteers during the sodium-loading (intravasal volume increase) protocol. Data are given as mean ± SEM and are expressed as the ratio between hormone concentration per µmol creatinine. †: significant (p < 0.05) difference in comparison with baseline values. §: significant (p < 0.05) difference in comparison with 10:00 values (before sodium infusion); Wilcoxon's matched pairs test.
- Fig. 7: Correlation analyses between urine flow and fractional excretion of sodium versus plasma and urinary hormone excretion of NT-proANP, NT-proBNP, and relaxin in healthy volunteers throughout the observation period. Spearman's rank correlation test calculated on the pooled data from 10 healthy volunteers during an observation period of ten hours after an infusion of 20 ml*kg⁻¹ isotonic saline.
UV; urine flow; FE_{Na}: fractional excretion of sodium; pl., plasma; ur., urine. Correlations are given as "Spearman's rho". m.s. analysis was not feasible due to missing samples. n.s. not significant. *: p<0.05
- Fig. 8: Frequency distribution of NT-proBNP levels (median) in blood donors (n=2948) at the age of 18-65 years (18-29 years, 30-39 years, 40-49 years, 50-59 years, 60-65 years). M, male; F, female.
- Fig. 9: Age group classified and gender-specific NT-proBNP levels in blood donors. N, number of blood donors. m, male; f, female.
- Fig. 10: Follow-up (12 month) of N=48 blood donors with elevated NT-proBNP levels.

- Fig. 11: NT-proBNP levels in blood donors and the relation to hemoglobin levels. m, male (diamonds); f, female (squares), t, total (triangles).
- Fig. 12: Age-group and gender-specific NT-proBNP levels (median) in blood donors in relation to creatinin levels. N, number of blood donors.
- Fig. 13: Characteristics of the study population of patients presenting with suspected cardiac disorders. t, total; m, male; f, female.
- Fig. 14: NT-proBNP levels in patients according to LVEF and NYHA classification.
- Fig. 15: NT-proBNP levels in males according LVEF.
- Fig. 16: NT-proBNP levels in females according LVEF.
- Fig. 17: Patients with NT-proBNP levels below cut-off (male: 84 pg/ml; female 155 pg/ml) with reduced LVEF.
- Fig. 18: NT-proBNP levels in patients with atrial fibrillation compared to patients without atrial fibrillation.
- Fig. 19: NT-proBNP levels in patients with myocardial infarct anamnesis (AMI) in comparison to patients without AMI anamnesis.
- Fig. 20: NT-proBNP levels in patients with angina pectoris in comparison to patients without angina pectoris
- Fig. 21: NT-proBNP levels in patients with elevated creatinin levels.
- Fig. 22: NT-proBNP levels in patients with regular thyroid function in comparison to patients with thyroid dysfunction.

- Fig. 23: NT-proBNP and BNP levels in a patient with septic infarction and subsequent pulmonary congestion. Hb, hemoglobin; Leukozyten, leukocytes,

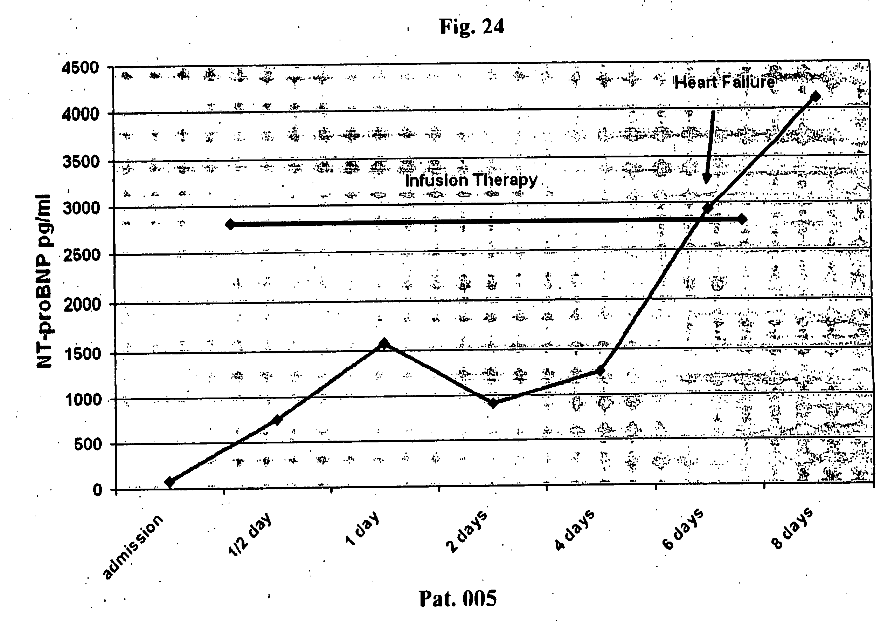

- Fig. 24: NT-proBNP levels in patient 005 of Example 8. Pat., patient.

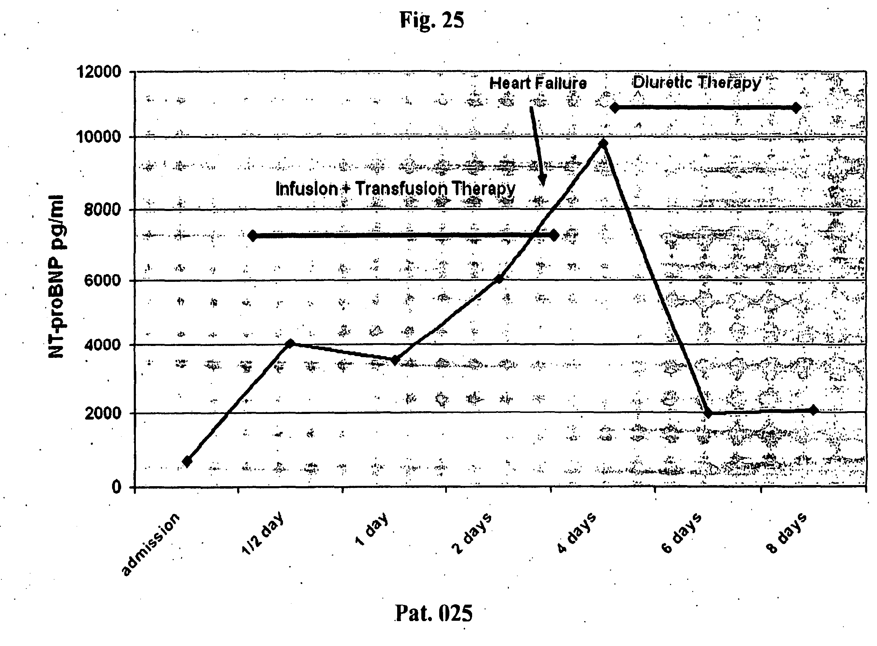

- Fig. 25: NT-proBNP levels in patient 025 of Example 8. Pat., patient.

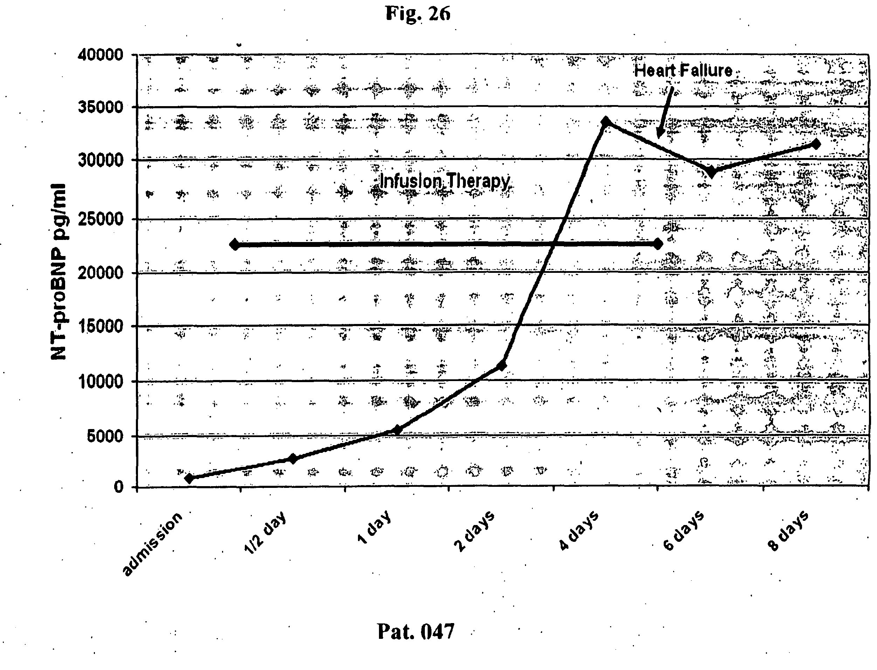

- Fig. 26: NT-proBNP levels in patient 047 of Example 8. Pat., patient.

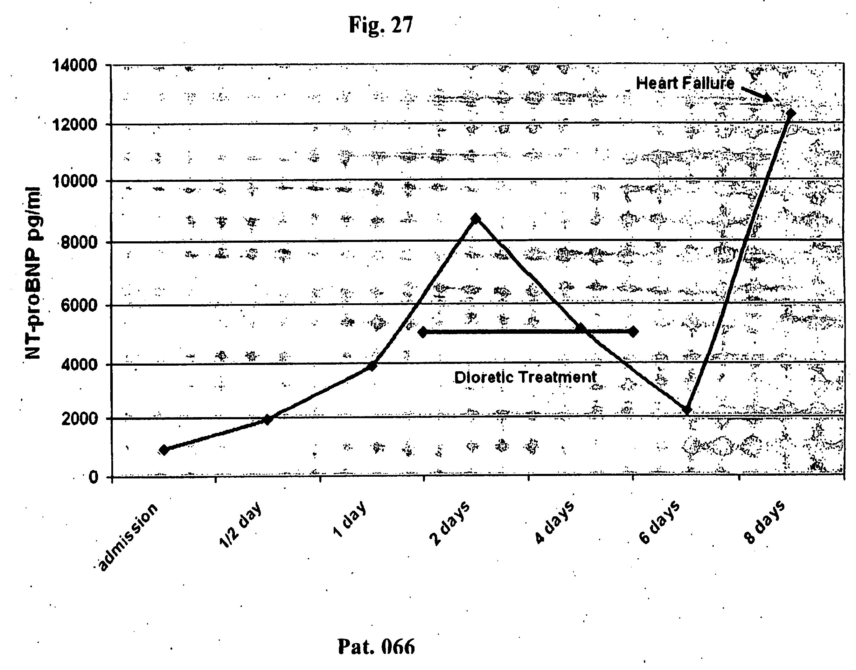

- Fig. 27: NT-proBNP levels in patient 066 of Example 8. Pat., patient.

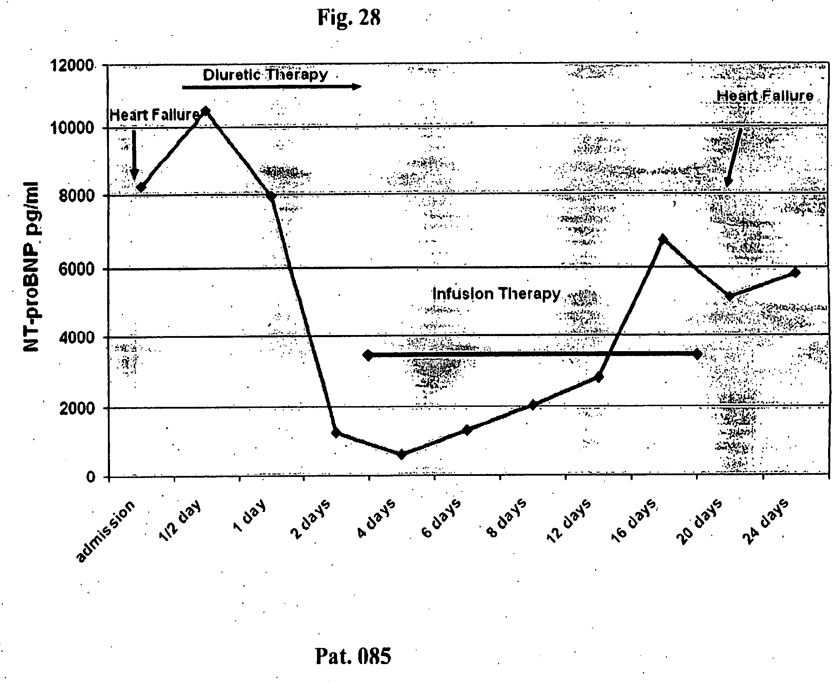

- Fig. 28: NT-proBNP levels in patient 085 of Example 8. Pat., patient.

The following examples illustrate the invention and are not intended to limit its scope in any way.

### Example 1

### Measurement of NT-proBNP:

NT-proBNP was determined by an electrochemoluminescence immunoassay (Elecsys proBNP sandwich immuno assay; Roche Diagnostics, Mannheim, Germany) on Elecsys 2010. The assay works according to the electrochemoluminescence sandwich immunoassay principle. In a first step, the biotin-labelled IgG (1-21) capture antibody, the ruthenium-labelled F(ab')₂ (39-50) signal antibody and 20 microliters of sample are incubated at 37 °C for 9 minutes. Afterwards, streptavidin-coated magnetic microparticles are added and the mixture is incubated for additional 9 minutes. After the second incubation, the reaction mixture is transferred to the measuring cell of the system where the beats are magnetically captured onto the surface of an electrode. Unbound label is removed by washing the measuring cell with buffer.

In the last step, voltage is applied to the electrode in the presence of a tri-propylamine containing buffer and the resulting electrochemoluminescent signal is recorded by a photomultiplier. All reagents and samples are handled fully automatically by the Elecsys® instrument. Results are determined via a calibration curve which is instrument-specifically generated by 2-point calibration and a master curve provided via the reagent barcode. The test was performed according to the instructions of the manufacturer.

### Example 2

Following approval by the Institutional Review Board and informed written consent, 16 healthy male, non-smoking volunteers (age: 27 ± 4 years; weight: 82 ± 11 kg; height: 184 ± 6 cm) on a customary sodium diet were studied. All subjects participated in the tilting protocol and 10 of the volunteers were additionally enrolled in the sodium loading protocol. The sodium loading protocol caused an increase in intravasal volume, a volume overload. The studies were performed in a temperature controlled laboratory after an overnight fast. After arrival at the laboratory at 8:00, subjects were placed in a supine position and equipped with a 16-gauge venous cannula allowing blood sampling without congestion. All subjects received a standard breakfast at 9:15 (2 slices of toast, marmalade, 3 ml *kg⁻¹ water).

### Tilting-protocol:

16 volunteers were randomly divided into two groups of n = 8 and studied in different body positions of 2 hours each: Following a resting period in the supine position, subjects were either tilted to a 15 ° head-down position (HD) or to the feet-down position (FD), were brought back into the supine position and afterwards tilted into the opposite direction.

Hemodynamics (heart rate (HR): electrocardiogram; mean arterial blood pressure (MAP: automated oscillomatric sphygmomanometer) were recorded every 15 min. and averaged for respective time periods. The endsystolic left atrial diameter (LAD) was determined by transthoracic echocardiography (ATL Ultrasound, Apogee; CX 100-150) in the long parasternal view. LAD measurements were performed after one hour in the respective body position and are given as the mean of 3 measurements. Blood for determination of blood chemistry and hormones was sampled every hour; beginning at 9:00.

### Sodium loading protocol:

10 volunteers were randomly studied during an observation period of 10 hours (control group) in the supine position or were subjected to an intravenous infusion of 15 ml *kg⁻¹ NaCl 0.9 % applied during 60 min. with an infusion pump from 10:00 to 11:00 (volume group). Studies were performed on different days at least two weeks apart.

In both groups, blood sampling for determination of blood chemistry and hormones as well as echocardiographic determinations of LAD were performed at 9:00, 10:00, 11:00, 12:00, 14:00, 16:00, and 18:00. In the volume group, urine was sampled by spontaneous voiding at 8:00, 10:00, 11:00, 12:00, 14:00, 16:00, and 18:00. Hemodynamics were determined every 15 min. and averaged for respective time periods.

### Analysis:

Blood for hormone analysis was sampled in EDTA-tubes containing 5000 U aprotinine (Trasylol, Beyer, Germany) and Lithium-Heparin-tubes (for clinical chemistry), as appropriate. Blood and urine samples were immediately spun for 10 min. at 3400 rpm at 4° C. Supernatants were stored at -80 °C until analysis.

Determination of NT-proANP: NT-proANP was determined by a competitive-binding radioimmuno assay with magnetic solid phase technique in a modification of Sundsfjord, J.A., Thibault, G., et al. (1988). Idenfication and plasma concentrations of the N-terminal fragment of proatrial natriuretic factor in man. J Clin Endocrinol Metab 66:605-10., using the same rabbit-anti-rat proANP polyclonal serum, human proANP (1-30) from Peninsula Lab (Bachem Ltd, St. Helene, UK) as the standard, and iodined, proANP 1-30 purified by HPLC for radio labelling. In order to achieve high sensitivity and good precision, Dynabeads M280 with sheep-anti-rabbit IgG (Dynal Biotech, Oslo, Norway) as solid phase and second antibody were used. The coefficient of variance, at 425, 1163, and 2490 pmol * l⁻¹ was 7.5, 3.7, and 3.4 %, respectively. The detection limit was 30 pmol/l.

### Determination of NT-proBNP:

NT-proBNP was determined by an electrochemoluminescence immunoassay (Elecsys proBNP sandwich immuno assay; Roche Diagnostics, Basel, Switzerland) on Elecsys 2010 (Mueller, T., Gegenhuber, A. (2003). Comparison of the Biomedica NT-proBNP enzyme immuno assay and the Roche NT-proBNP chemiluminescence immuno assay: implications for the prediction of symptomatic and asymptomatic structural heart disease. Clin. Chem. 49:976-9), see also Example 1. The mean intra-assay variance was 4.3 % (range: 2.7 to 5.9 % for plasma samples with a concentration between 7.6 to 2732 pmol *l⁻¹ with an interassay variance of 3.2 %. The lower detection limit was 0.6 pmol*l⁻¹.

### Statistical analyses:

Significance was set to p < 0.05.

Tilting-protocol: With respect to variance at baseline, hormone and LAD data were normalized according to baseline levels (set to 100 %) and analyzed by ANOVA for repeated measures. Bonferoni correction was not used. Intra-individual differences in comparison with normalized baseline values were analyzed with paired Student's t-test.

Sodium-loading-protocol: Intra-individual differences between the control and the volume group were analyzed by Friedman's test followed by Wilcoxon's matched pairs test. With respect to the number of measurements and the stample size, Bonferoni correction was not used. Intra-individual differences during the observation period were determined by Wilcoxon's matched pairs test. For correlation analyses Spearman's rank correlation test was used.

### Results of the tilting-protocol:

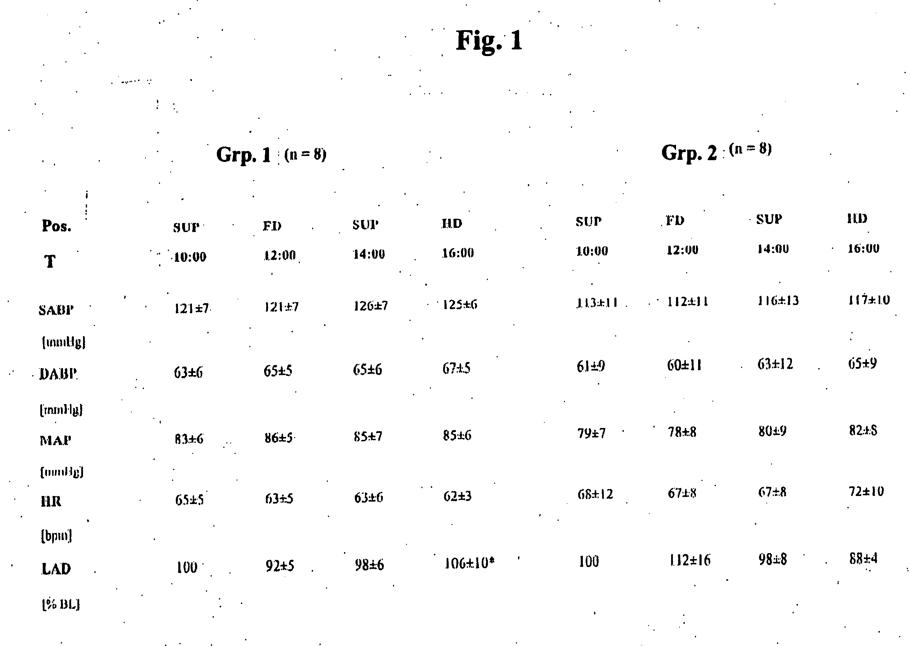

Hemodynamics: Neither heart rate nor arterial blood pressure showed significant intra- or inter-individual changes throughout the observation period (Fig. 1). A significant difference in LAD was observed during the second tilting period (Fig. 1).

Clinical Chemistry: Plasma sodium, potassium, creatinine at baseline were not different between both tilting groups (plasma sodium: group I: 138 ± 2 mmol *l⁻¹; group II: 139 ± 2 mmol *l⁻¹; plasma potassium: group I: 3.8 ± 0.3; group II: 3.6 ± 0.2 mmol *l⁻¹; plasma creatinine: group I: 75 ± 9 µmol *l⁻¹; group II: 81 ± 8 µmol *l⁻¹. No significant between-group-variations in these parameters were observed throughout the observation period.

The course of normalized levels of NT-proANP and NT-proBNP is depicted in Fig. 2. Additionally shown are levels of relaxin (RLX). NT-proANP levels were higher during the HD than during FD in the second tilting period. NT-proBNP levels increased over time until 15:00 but were not different between both groups. No significant inter-individual variations were observed in plasma RLX levels. The intra-individual course of this hormone was comparable in both groups, showing a search in normalized RLX levels at 15:00 in comparison with baseline and an increase from 15:00 to 16:00 back to baseline levels.

### Results of the volume-loading protocol:

Hemodynamics: No significant within-and-between-group differences were observed in HR and MAP (Fig. 3). A small albeit significant increase in LAD was observed after fluid loading from 11:00 to 12:00. Thereafter, LAD decreased back to baseline levels (Fig. 3).

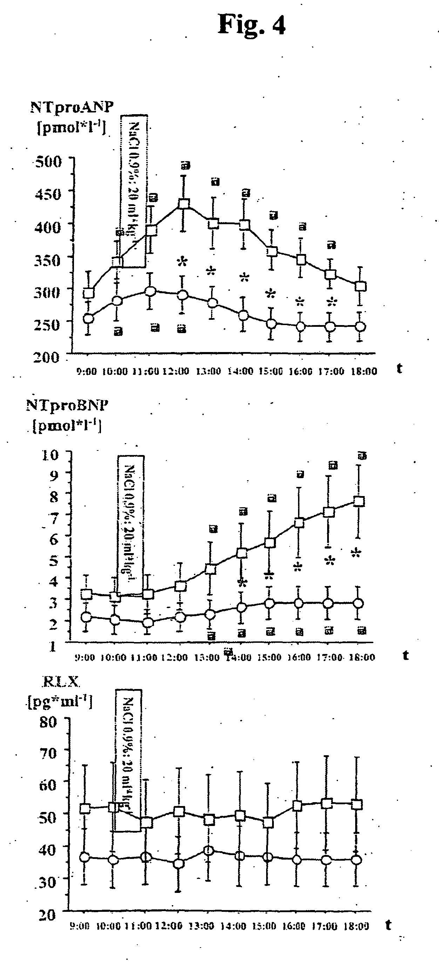

Clinical chemistry: plasma sodium, potassium, creatinine and hematocrit at baseline were not different between the control and the sodium-loading protocol (plasma sodium: control: 139 ± 2 mmol *l⁻¹; sodium-loading: 140 ± 2 mmol *l⁻¹; plasma potassium: control: 3.6 ± 0.3 mmol *l⁻¹; sodium-loading: 3.4 ± 0.7 mmol *l⁻¹; plasma creatinine: control: 80 ± 11 µmol *l⁻¹; sodium-loading: 76 ± 9 µmol *l⁻¹; hematocrit: control: 40.7 ± 2.0 %; sodium-loading: 40.1 ± 1.8 %). Plasma hormone levels: the plasma levels of NT-proANP, NT-proBNP, and RLX are depicted in Fig. 4. In the sodium-loading group, NT-proANP increased immediately after sodium-loading with a peak 2 hours after the infusion. A moderate increase was also observed in the control group. However, after 12:00, NT-proANP levels in this group were not different from baseline levels. NT-proBNP showed a protracted increase up to the end of the observation period in both groups. NT-proBNP levels in the sodium infusion group were significantly higher than in the control group after 13:00. No significant variations or between-group differences in RLX levels were observed.

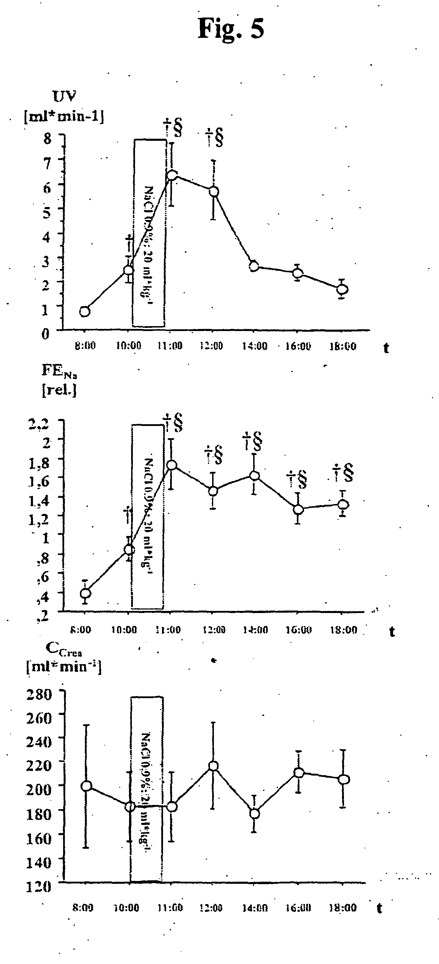

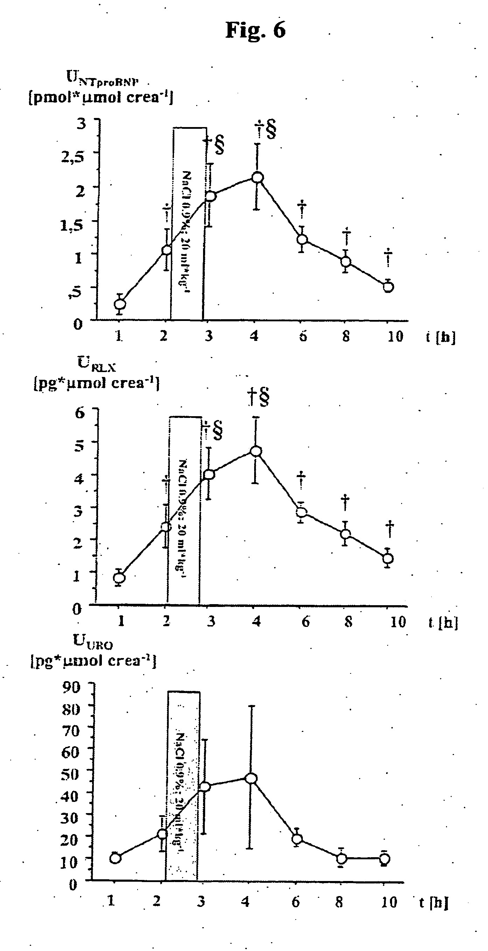

Renal function parameters: urine flow and fractional sodium excretion showed a moderate increase from 8:00 to 10:00. After sodium infusion, a further and more pronounced increase was observed (Fig. 5). Thereafter, UV increased back to pre-infusion levels while FE_{Na} remained elevated until the end of the observation period. Creatinine clearance did not change throughout the observation period (Fig. 5). Urinary excretion of hormones: urinary excretion of NT-proBNP, RLX, and urodilatin is given in Fig. 6. Measurement of NT-proANP was only possible in a minority of urine samples; hence no calculations on this parameter were performed. U_{NT-proBNP} and U_{RLX} increased significantly from 8:00 to 10:00 and further to reach a peak at 12:00. Thereafter, urinary hormone excretion decreased but remained elevated above baseline levels. U_{URO} showed a comparable course like U_{NT-proBNP} and U_{RLX}, however, due to a high variance and the fact, that U_{URO} concentrations in tool subjects were below the detection limit, the course of this peptide after infusion did not reach statistical significance. However, U_{URO} levels at 14:00 were significantly higher than baseline levels.

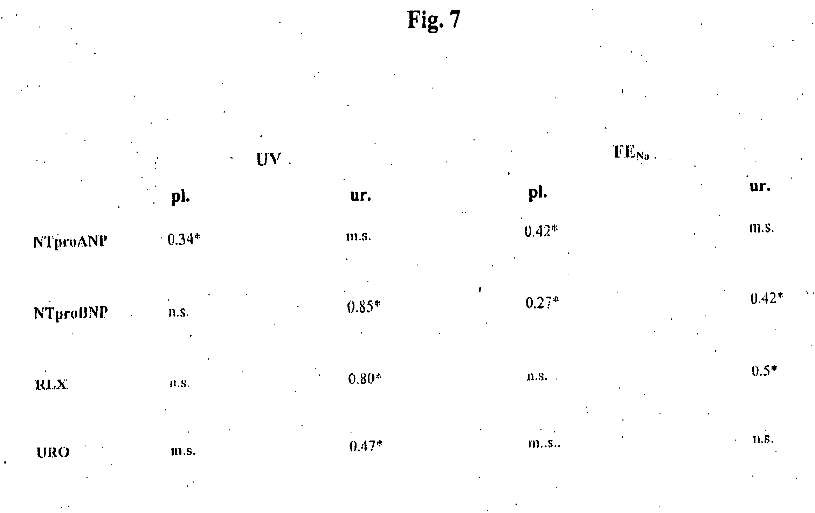

Correlation analyses: correlation analyses of plasma hormone levels at baseline between the control and the sodium-loading group for determination of the reliability of the measurements revealed the following significant correlations: NT-proANP: rho = 0.91; NT-proBNP: rho = 0.82; RLX: rho = 0.91. Correlation analyses between urinary functional parameters and hormonal plasma levels and urinary excretion of these hormones are given in Fig. 7. These analyses reveal minor relationships between the plasma levels of NT-proANP and NT-proBNP on one hand and FE_{Na} on the other hand. However, better correlations were observations between the urinary excretion of NT-proBNP, RLX, and URO and UV and between NT-proBNP and RLX and FE_{Na}.

### Example 3

### A study of NT-proBNP levels in blood donors:

A total of 1981 blood donors were recruited from the blood transfusion service of the University of Mainz, Germany. The majority of the blood donors were repeat donors and repeat donors do receive a physical examination at yearly interval. Based on this examination all blood donors included into the study were considered clinically healthy. At the time of blood donation hemoglobin levels as well as creatinin levels were taken. All determinations were done before blood donation. The study was conducted according to the Declaration of Helsinki and was approved by a local ethical committee.

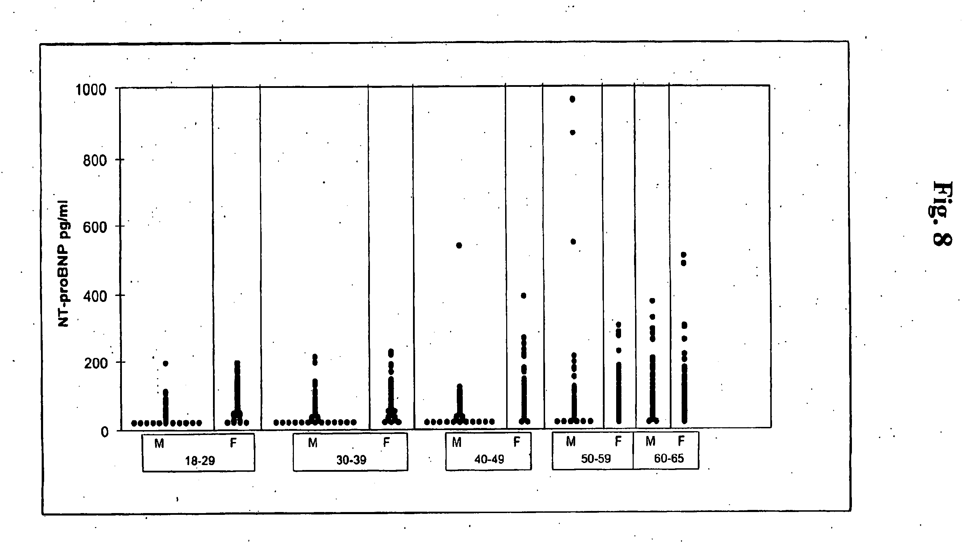

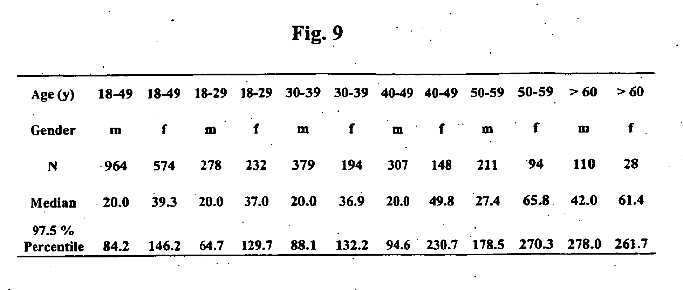

As depicted in Fig. 8 individual NT-proBNP values are plotted in relation to age and sex. As becomes evident from Fig. 7, NT-proBNP levels (median) were higher in women than in men. Outliers were more frequently observed in elderly individuals (above the age of 50 years) whereas in younger individuals (below 50 years of age) individual determinations clustered. Age and sex-related reference values based on the 97.5 perentile were calculated and found to be 84.2 pg/ml for males and 146.2 pg/ml for females respectively under the age of 50 years (Fig. 9).

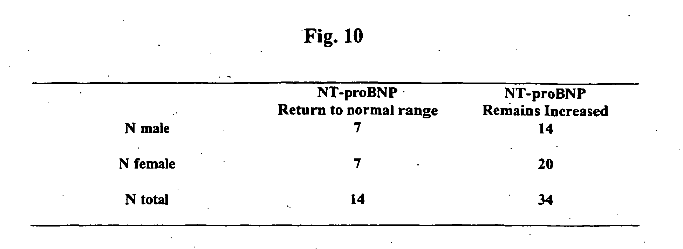

A second sample at an approximately 12 months interval was collected from all individuals who were outside the above range as can be seen from table Fig. 10, the majority of samples remained outside the respective reference range suggesting that these elevated values were constant findings. A small subset of individuals with initial values outside the range described in the second sample had values that were considered to be within the defined reference ranges.

In order to assess whether NT-proBNP values were independent on hemoglobin levels, hemoglobin concentrations were determined in males and females and found to be in average 1.5 g/ml lower in females than in males (Fig. 10). Hemoglobin levels did not depend on age.

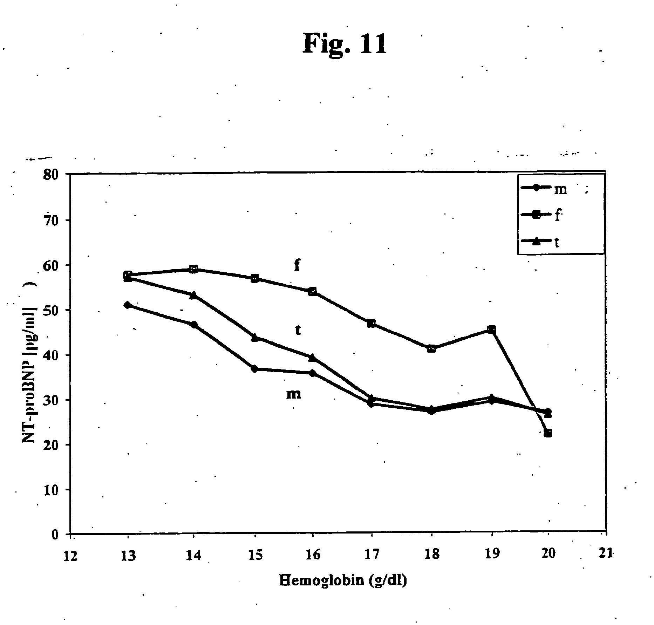

When NT-proBNP values were compared between males and females at the same hemoglobin levels and in age-matched groups there was still a difference between males and females in terms of NT-proBNP levels suggesting that hemoglobin levels did not explain the different concentrations found for NT-proBNP between males and females. It also became apparent that NT-proBNP levels were in fact hemoglobin-dependent, NT-proBNP levels increased with decreasing hemoglobin concentration (Fig. 11).

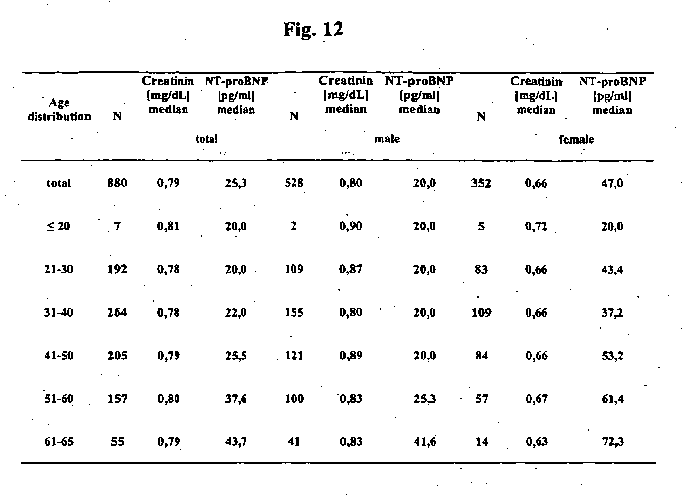

In a subset of individuals creatinin levels were compared to NT-proBNP levels. In the group studied creatinin levels were in the normal range for all individuals tested. Creatinin levels did not increase with age, in contrast, NT-proBNP levels increased with age suggesting that kidney function might not trigger increase of NT-proBNP with increasing age (Fig. 12).

The study was initiated to determine normal and reference NT-proBNP values in an apparently healthy population. As shown, individual NT-proBNP levels clustered up to the age of 50 years with only few outliers. This finding is consistent with the assumption that cardiac and specifically cardiovascular diseases are rare in this age group, therefore values obtained in individuals below the age of 50 were considered based on a 97.5 percentile as normal values. These values were also found to be different between males and females. It could also be shown that in fact hemoglobin levels affected the level of NT-proBNP in that individuals with lower hemoglobin had higher NT-proBNP levels. When looking at the same hemoglobin levels there were still differences between men and women. Thus, hemoglobin levels did not explain for the differences in NT-proBNP levels seen between both sexes.

This study showed that a substantial number of individuals had NT-proBNP levels exceeding the 97.5 percentile of individuals below the age of 50. The number of these outliers increased with age. Determination of NT-proBNP levels was done by the Elecsys® immunoassay as described in Example 1.

### Example 4

### A Study of NT-proBNP levels in patients presenting with suspected cardiac disorders:

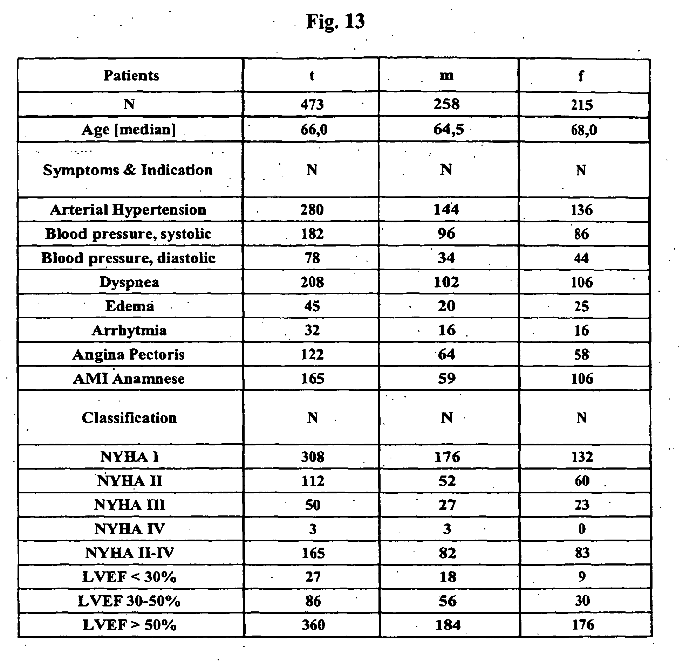

A total of 473 patients presenting to 18 cardiologists were recruited for the study. They received a medical history, a physical examination and an echocardiogram where left ventricular ejection fraction was recorded. In addition, 10 ml of blood was drawn, centrifuged and stored at -20 °C until analyzed. Major demographic variables of the patients included in this study are depicted in Fig. 13. The study was approved by a local ethical committee and conducted according to the Declaration of Helsinki.

The following tests were done in all or the majority of the patients: Creatinin levels, TSH, FT4, and NT-proBNP. The tests were conducted according to the instructions of the manufacturer (Roche Diagnostics, Mannheim, Germany). NT-proBNP was analyzed using a newly developed immunoassay (Roche Diagnostics, Mannheim, Germany) using an Elecsys® 2010 instrument (see Example 1).

Significancies were calculated based on Wilcoxon Score method and Pearson Chi-Square test: Significance is present at p-values **P* < 0.05, ***P* < 0.01, *** *P* < 0.001. The probability of error should not exceed 5 %.

Patients were separated into three groups according to left ventricular injection fraction (LVEF), namely under 30 % LVEF, 30-50 % LVEF, and over 50 % LVEF. The patients were also graded according to NYHA classification in grade I-IV.

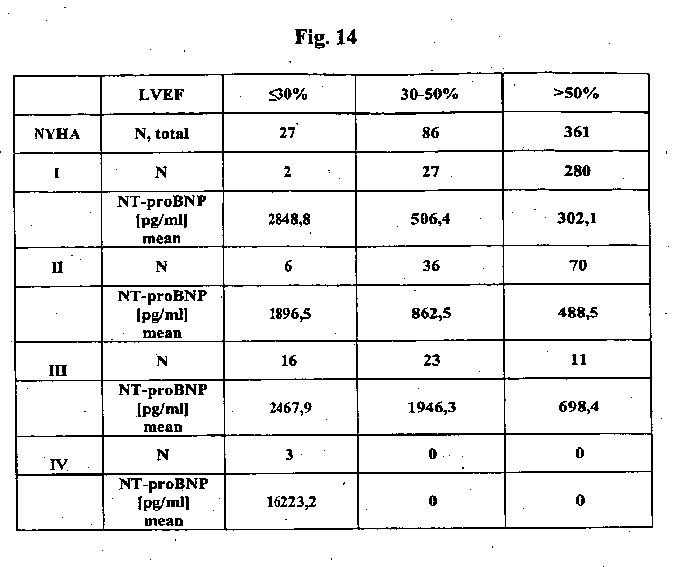

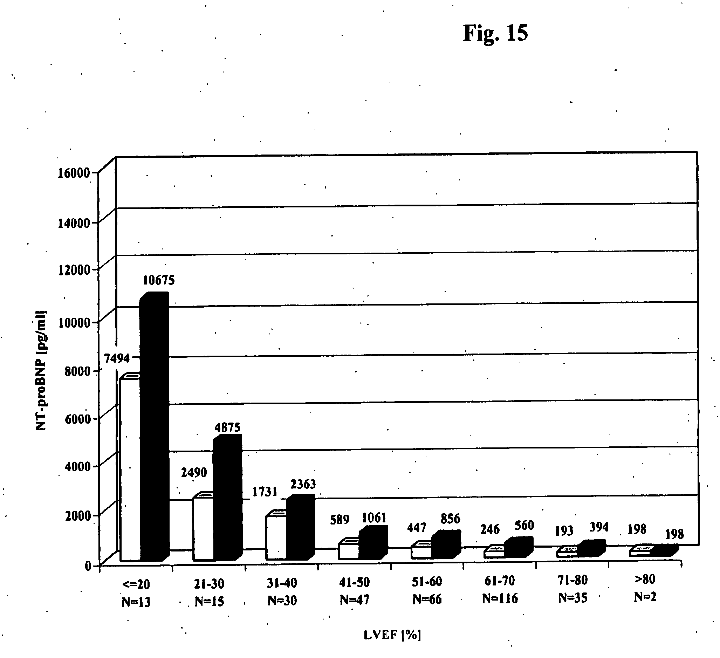

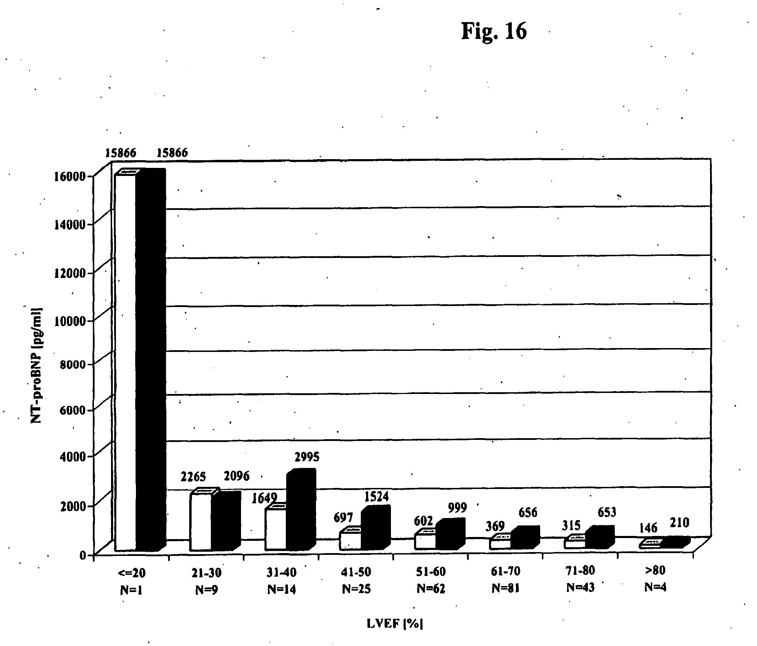

As depicted in Fig. 14, NT-proBNP levels were recorded based on the level of left ventricular ejection fraction and based on symptoms. The majority of individuals had increased NT-proBNP levels if a cut-off of 84 pg/ml for males and 146 pg/ml for females were used, this discriminates between normal and abnormal cardiac function (see Example 1). The mean NT-proBNP levels increased with the level of symptoms as assessed by NYHA classification and with the level of impaired ejection fraction as measured by echo. The dependency of NT-proBNP on left ventricular injection fraction is also summarized in Fig. 15 and 16 for males and females respectively. As can be seen from the figures, NT-proBNP levels (median) increased with decreasing ejection fraction.

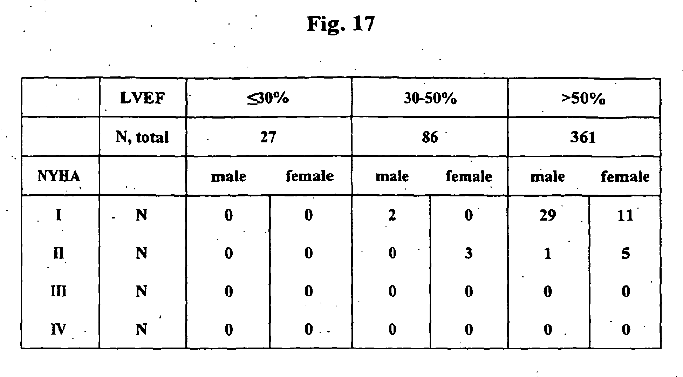

As shown in Fig. 17, only a minority of individuals recruited for the study in the cardiologists centers had normal NT-proBNP levels based on cut-offs made from a study in blood donors below the age of 50 (see Example 3). Normal NT-proBNP values clustered in individuals with unimpaired left ventricular fraction and without symptoms, only few outliers were identified.

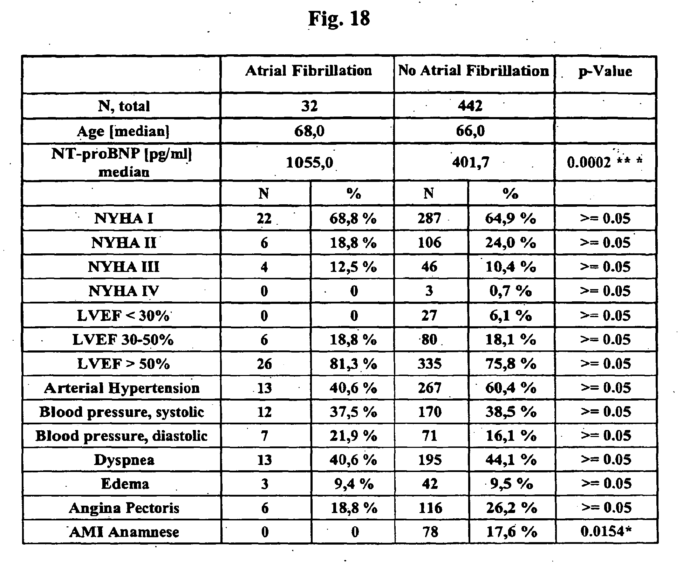

A total of 32 individuals had atrial fibrillation as indicated by electrocardiogram (ECG) while the majority of individuals had no evidence of atrial fibrillation. As can be seen from Fig. 18, median values in the atrial fibrillation group were higher than in the non-atrial fibrillation group. Major demographic valuables for these patient groups are depicted. Individuals who had no atrial fibrillation had more frequently a history of myocardial infarction and Angina Pectoris. The data suggest that atrial fibrillation represents an independent contributor for elevated NT-proBNP levels (P: 0.0002).

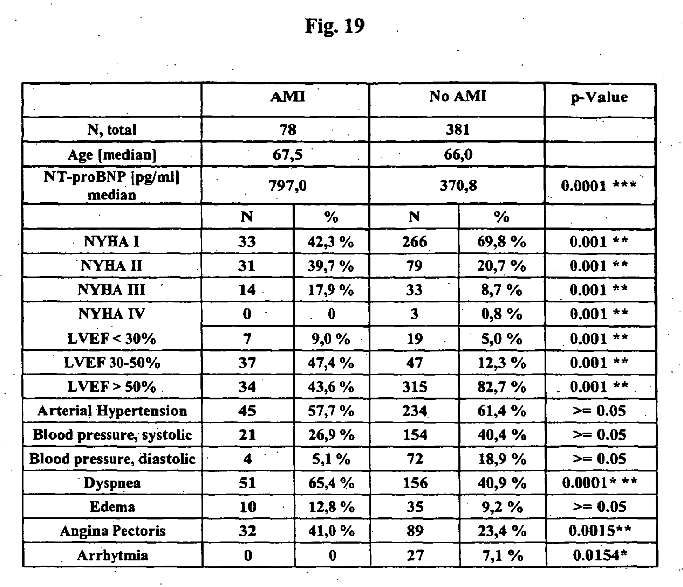

A total of 78 individuals had a history of myocardial infarction (MI) while the majority had no history of MI. Individuals with the history of myocardial infarction had higher NT-proBNP levels than those who had no history of MI (Fig. 19).

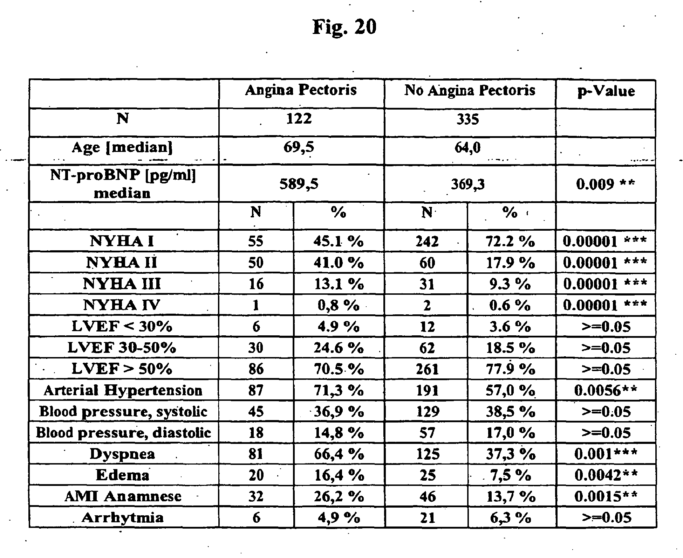

NT-proBNP values were higher in individuals with a history of angina pectoris than in those who had no history of angina pectoris (Fig. 20). Patients with a history of angina pectoris were not frequently symptomatic, had more frequently heart diseases and more frequently of history of myocardial infarction (Fig. 19).

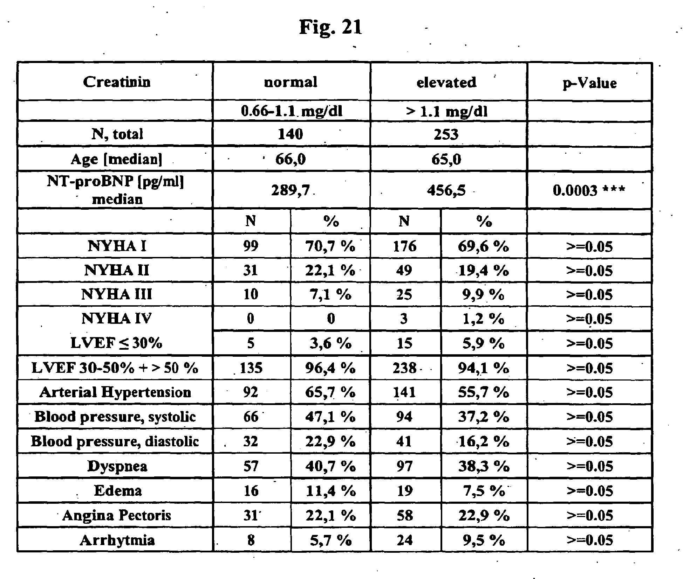

Creatinin was determined in 470 individuals. Only 152 individuals had creatinin levels in the normal range, 318 were outside of the normal range. Individuals with elevated creatinin levels had higher NT-proBNP levels than those with normal creatinin levels. Demographic variables suggest that individuals with elevated creatinin levels had more frequently a history of myocardial infarction. The data suggest that impaired kidney function per se might contribute the elevation of NT-proBNP levels when patients with a history of MI (AMI) were excluded from assessment (Fig. 21).

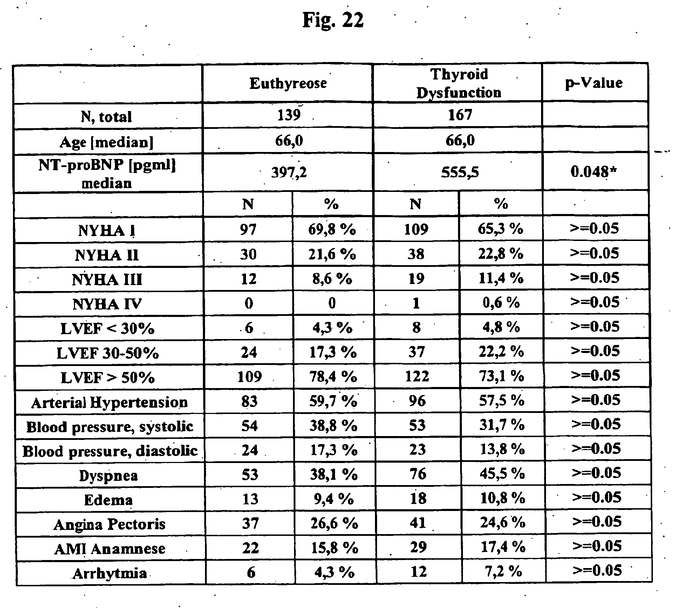

In a subgroup of 306 individuals thyroid function was measured. Based on TSH and FT4 levels the patients were classified in individuals with normal thyroid function and in those with abnormal thyroid function. The majority of the individuals with abnormal thyroid function had elevated TSH levels, but normal FT4, suggesting compensated hypothyroid function. Median NT-proBNP levels were higher in individuals with abnormal thyroid function than in those with normal thyroid function. This suggest that thyroid dysfunction represents a contributor to elevated NT-proBNP levels most likely associated with impaired cardiac function through impaired thyroid function (Fig. 22).

The present data suggest that when compared to data obtained in blood donors (Example 3) the majority of patients presenting to cardiologists has elevated NT-proBNP levels. NT-proBNP levels increased with levels of symptoms and with impairment of left ventricular ejection fraction. The fact that elevated NT-proBNP levels were recorded in asymptomatic individuals and in individuals with unimpaired ejection fraction indicates that NT-proBNP recognizes cardiac complication earlier than current gold standard methodology used by cardiologists. In the present study it was found that kidney function was frequently impaired based on creatinin levels in a group of patients with evidence of cardiac complication. This is in contrast to a study in blood donors where significantly lower and normal creatinin levels were found in a population of similar age (see Example 3). The study suggests that both components, kidney function and cardiac complication, need to be considered, and the data also indicate that mild to moderate renal dysfunction does not influence the interpretation of NT-proBNP values in the diagnosis and assessment of cardiac complication.

The data also indicate that thyroid dysfunction might be associated with cardiac dysfunction and might contribute to elevated NT-proBNP levels.

### Example 5

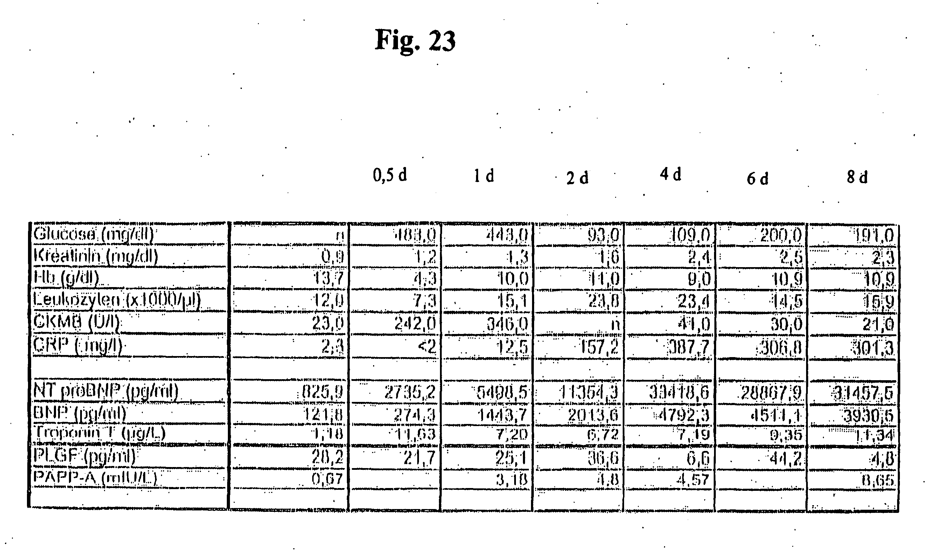

A 76-year old female patient with an acute septic event was hospitalized (Fig. 23). During the course of the disease, the patient developed fever and required large volume infusions as well as antibody therapy. Nevertheless the patient developed a septic shock and the blood pressure fell although she received infusion treatment. During the course of the disease CRP and NT-proBNP gradually increased, the latter as a sign of fluid overload and progressive heart failure.

### Example 6

A 70-year-old man with a known history of type II-diabetes and known coronary heart disease presents at his general practitioner. He complains of pain in the region of his right knee. The pain causes particular discomfort during climbing of stairs. At the time of presentation no dyspnea, which is a sign of a manifest heart sufficiency, is evident. Having performed an X-ray examination of the knee, the general practitioner considers the administration of non-steroid anti-rheumatics. The NT-proBNP value, which was determined at first presentation, amounts to 1800 pg/ml, indicating an asymptomatic cardiac complication. The treatment with non-steroid anti-rheumatics is initiated at simultaneous administration of cardiac medication and at close clinical surveillance and following measurements of NT-proBNP.

### Example 7

A 76-year-old female patient with operable colon carcinoma without significant history of other diseases is admitted to a surgical ward. The value of NT-proBNP is 1200 pg/ml. The colon carcinoma is removed surgically. Subsequently parenteral nutrition is initiated until resumption of intestinal function. The need of liquid amounts to 3000 ml per day. At careful equilibration during subsequently positive equilibration, a treatment with diuretics is initiated to re-establish an equilibrated water balance.

### Example 8

A total of 120 patients in an intensive care unit were diagnosed in regular intervals according to standard criteria. NT-proBNP was analysed retrospectively. In connection with infusion therapy and/or termination of treatment with diuretics, an increase of NT-proBNP was observed a total of 5 patients with subsequent clinical diagnosis of cardiac insufficiency. Increased levels of NT-proBNP were also observed before initiating infusion therapy, indicating cardiovascular risk.

Patient 005: 45 year-old patient with known coronary heart disease and pneumonia. The NT-proBNP level began to increase on day 4 and cardiac insufficiency was diagnosed on day 6.

Patient 025: 66 year-old patient with status after myocardial infarction and anemia with infusions/transfusions briefly after hospitalization. The NT-proBNP level began to increase between day 1 and day 2. Pulmonary edema was observed on day 3 and the patient was treated with diuretics.

Patient 047: 76 year-old pateint with known angina pectoris, known coronary heart disease and exsiccosis (dehydration) at the time of admission to the hospital. Exsiccosis was treated with approximately 2 liters per day. NT-proBNP increased continuously, diagnosis of cardiac insufficiency on day 5 after hospitalization.

Patient 066: A 64 year-old female patient with known three-vessel-disease and verified coronary heart disease, suffering from hypercholesterolemia, depression and anemia. Aggravation after the first day and treatment with diuretics until day 5. Subsequently increase of NT-proBNP until day 8 and manifestation of cardiac insufficiency as a sign of a rebound of cardiac insufficiency with volume overload.

Patient 085: 78 year-old patient with cardiac insufficieny followed by treatment with diuretics and decrease of the NT-proBNP level (at high start levels). Subsequently infusion treatment in the context of nutrition (approximately 2.5 liters/day). Increase of the NT-proBNP level on day 12, diagnosis of cardiac insufficiency on day 18.

## Claims

1. A method for diagnosing the risk of a patient, who shows no symptoms of a cardiovascular disease according to the NYHA classification and who has no history of cardiovascular complication, of suffering from a cardiovascular complication as a consequence of a future increase intravasal volume, comprising the steps of
a) measuring, in vitro, the level of a natriuretic peptide from the group of ANP, or NT-proANP and/or BNP, or NT-proBNP
b) diagnosing the risk of the patient by comparing the measured level to at least one known level(s) associated with different grades of risk in a patient.

2. The method according to claim 1, wherein the natriuretic peptide is NT-proBNP.

3. The method according to claim 1 or 2, wherein a plasma level of more than 60 and less than 1000 pg/ml of NT-proBNP in a male patient is associated with an increased risk of suffering from a cardiovascular complication, wherein said increased risk indicates that treatment may be adapted.

4. The method according to claim 1 or 2, wherein a plasma level of more than 120 and less than 1000 pg/ml of NT-proBNP in a female patient is associated with an increased risk of suffering from a cardiovascular complication, wherein said increased risk indicates that treatment may be adapted.

5. The method according to claim 1 or 2, wherein a plasma level from 1000 to 5000 pg/ml of NT-proBNP is associated with a highly increased risk of suffering from a cardiovascular complication, wherein said highly increased risk indicates that it may be reconsidered whether any intravasal volume increase can be tolerated.

6. The method according to claim 1 or 2, wherein a plasma level of more than 5000 pg/ml of NT-proBNP is associated with a very highly increased risk of suffering from a cardiovascular complication, wherein said very highly increased indicates that it may be reconsidered whether any intravasal volume increase can be tolerated and wherein also immediate hospitalization and/or intensive cardiac treatment may be considered.

7. The method according to any of claims 1 to 6, wherein the increase of intravasal volume is caused by disease, particularly by sepsis or gammopathy.

8. The method according to any of claims 1 to 7, wherein the increase of intravasal volume is caused artificially, by a liquid to be administered by infusion or transfusion, or by a drug to be administered.

9. The method according to claim 8, wherein the drug to be administered is chosen from the group consisting of
a) non-steroidal anti-rheumatics
b) corticosteroids,
c) diabetes drugs,
d) estrogens,
e) TNF inhibitors,
f) selective Cox-2 inhibitors

10. The method according to any of claims 1 to 9, wherein the cardiovascular complication is coronary heart disease, stable angina pectoris, acute coronary syndrome, wastable angina pectoris, myocardial infarction; left ventricular dysfunction, congestive heart failure, or pulmonary congestion.

11. The method according to claim 10, wherein the cardiovascular complication is pulmonary congestion.

12. The method acceding to any of claims 1 to 11, wherein the level of the cardiac hormone is measured using a specifically binding ligand, an array, a microfluidic device, a chemiluminescence analyzer, or a robotic device,

13. The method according to claim 12, wherein the specifically binding ligand is an antibody or an aptamer.

14. The method according to any of claims 1 to 13, wherein the specifically binding ligand is labeled.

15. Use of a diagnostic means capable of measuring, in vitro, a patient's level of a natriuretic peptide from the group of ANP, NT-proANP, and/or BNP or NT-proBNP for diagnosing the risk of the patient, who shows no symptoms of a cardiovascular disease according to the NYHA classification and who has no history of cardiovascular complication, of suffering from a cardiovascular complication as a consequence of a future increase of intravasal volume.

16. A method of deciding about in the future administering to a patient who shows no symptoms of a cardiovascular disease according to the NYHA classification and who has no history of cardiovascular complication, an infusion, a transfusion, or a drug causing volume overload comprising
a) measuring, in vitro, the level of a natriuretic peptide from the group of ANP, NT-proANP and/or BNP, NT-proBNP in the patient,
b) comparing the measured level with at least one known level(s) associated with different grades of risk in a patient,
c) optionally initiating an examination of the patient by a cardiologist,
d) recommending or refraining the administering the infusion, transfusion, or drug, optionally in consideration of the result of the parent's examination by the cardiologist.

17. The method according to claim 16, wherein the method is for deciding about in the future administering to a patient, who shows no symptoms of cardiovascular complication, a drug causing volume overload and wherein the drug is a selective Cox-2 inhibitor.

## Patentansprüche

1. Verfahren zur Diagnose des Risikos eines Patienten, der keine Symptome einer kardiovaskulären Erkrankung gemäß der NYHA-Klassifikation zeigt und der keine Vorgeschichte kardiovaskulärer Komplikationen hat, als Folge eines künftigen Anstiegs des intravasalen Volumens kardiovaskuläre Komplikationen zu erleiden, bestehend aus den Schritten
a) in-vitro-Bestimmung des Spiegels eines natriuretischen Peptids aus der Gruppe von ANP oder NT-proANP und/oder BNP oder NT-proBNP,
b) Diagnose des Risikos des Patienten durch den Vergleich des gemessenen Spiegels mit wenigstens einem bekannten Spiegel, der mit unterschiedlichem Risiko eines Patienten assoziiert ist.

2. Verfahren nach Anspruch 1, wobei das natriuretische Peptid NT-proBNP ist.

3. Verfahren nach Anspruch 1 oder 2, wobei ein Plasmaspiegel von mehr als 60 und weniger als 1000 pg/ml NT-proBNP eines männlichen Patienten mit einem erhöhten Risiko des Erleidens einer kardiovaskulären Komplikationen assoziiert ist und anzeigt, dass die Behandlung möglicherweise angepasst werden muss.

4. Verfahren nach Anspruch 1 oder 2, wobei ein Plasmaspiegel von mehr als 120 und weniger als 1000 pg/ml NT-proBNP eines weiblichen Patienten mit einem erhöhten Risiko des Erleidens einer kardiovaskulären Komplikationen assoziiert ist und anzeigt, dass die Behandlung möglicherweise angepasst werden muss.

5. Verfahren nach Anspruch 1 oder 2, wobei ein Plasmaspiegel von 1000 bis 5000 pg/ml NT-proBNP mit einem stark erhöhten Risiko des Erleidens einer kardiovaskulären Komplikationen assoziiert ist und wobei dieses stark erhöhte Risiko anzeigt, dass überdacht werden muss, ob ein weiterer Anstieg des intravasalen Volumens toleriert werden kann.

6. Verfahren nach Anspruch 1 oder 2, wobei ein Plasmaspiegel von mehr als 5000 pg/ml ProBNP mit einem sehr stark erhöhten Risiko des Erleidens kardiovaskulärer Komplikationen assoziiert ist, und wobei der besagte sehr stark erhöhte Spiegel anzeigt, dass überdacht werden muss, ob ein weiterer Anstieg des intravasalen Volumens toleriert werden kann, und wobei die sofortige Einweisung in ein Krankenhaus und/oder kardiologische Intensivbehandlung überdacht werden müssen.

7. Verfahren nach einem der Ansprüche 1 bis 6, wobei der Anstieg des intravasalen Volumens durch eine Krankheit, insbesondere durch Sepsis oder Gammopathie, verursacht wird.

8. Verfahren nach einem der Ansprüche 1 bis 7, wobei der Anstieg des intravasalen Volumens künstlich durch eine per Infusion oder Transfusion zu verabreichende Flüssigkeit oder von einem zu verabreichenden Medikament verursacht ist.

9. Verfahren nach Anspruch 8, wobei das zu verabreichende Medikament aus folgender Gruppe ausgewählt wurde:
a) nichtsteroidale Antirheumatika
b) Kortikosteroide
c) Antidiabetika
d) Östrogene
e) TNF-Inhibitoren
f) Selektive COX-2 Inhibitoren

10. Verfahren nach den Ansprüchen 1 bis 9, wobei die kardiovaskuläre Komplikation eine koronare Herzkrankheit, stabile Angina Pectoris, akutes Koronarsyndrom, instabile Angina Pectoris, Myokardinfarkt, linksventrikuläre Dysfunktion, eine Herzinsuffizienz oder ein Lungenstauung ist.

11. Verfahren nach Anspruch 10 , wobei die kardiovaskuläre Komplikation eine Lungenstauung ist.

12. Verfahren nach einem der Ansprüche 1 bis 11, wobei der Spiegel des kardialen Hormons unter Benutzung eines spezifisch bindenden Liganden, eines Arrays, eines auf Mikrofluidität beruhenden Geräts, eines Analysegeräts zur Chemolumineszenzmessung oder eines automatisierten Geräts bestimmt wird.

13. Verfahren nach Anspruch 12, wobei der spezifisch bindende Ligand ein Antikörper oder Aptamer ist.

14. Verfahren nach einem der Ansprüche 1 bis 13, wobei der spezifisch bindende Ligand markiert ist.

15. Verwendung einer diagnostischen Vorrichtung ermöglichend die in-vitro-Bestimmung des Spiegels eines natriuretischen Peptids aus der Gruppe von ANP, NT-proANP und/oder BNP oder NT-proBNP bei einem Patienten, der gemäß der NYHA-Klassifikation keine Symptome einer kardiovaskulären Erkrankung zeigt und der keine Vorgeschichte kardiovaskulärer Komplikationen hat, zur Diagnose des Risikos dieses Patienten, eine kardiovaskuläre Komplikation als Folge eines künftigen Anstiegs des intravasalen Volumens zu erleiden.

16. Verfahren zur Entscheidung über die künftige Verabreichung einer Infusion, Transfusion oder eines Hypervolämie auslösenden Medikaments an einen Patienten, der kein Symptom einer kardiovaskulären Erkrankung nach der NYHA-Klassifikation zeigt und der keine Vorgeschichte kardiovaskulärer Komplikationen hat,enthaltend:
a) in-vitro-Bestimmung des Spiegels eines natriuretischen Peptids aus der Gruppe von ANP, NT-proANP und/oder BNP, NT-proBNP,
b) Vergleich des gemessenen Spiegels mit wenigstens einem bekannten Spiegel, der/die mit unterschiedlichen Risiken des/der Patienten assoziiert sind,
c) optional Einleiten einer Untersuchung des Patienten durch einen Kardiologen,
d) Empfehlung oder Ablehnung einer Infusion, Transfusion oder eines Medikaments, optional unter Berücksichtigung der Ergebnisse einer Untersuchung des Patienten durch den Kardiologen.

17. Verfahren nach Anspruch 16, wobei die Methode zur Entscheidung der zukünftigen Verabreichung eines Medikaments verursachend Hypervolämie in einem Patienten dient, der keine Symptome einer kardiovaskulären Komplikation zeigt, wobei dieses Medikament ein selektiver Cox-2-Inhibitor ist.

## Revendications

1. Procédé pour diagnostiquer le risque auquel est exposé un patient, qui ne présente aucun symptôme d'une maladie cardio-vasculaire conformément à la classification NYHA et qui n'a pas d'histoire de complications cardio-vasculaires, de souffrir d'une complication cardio-vasculaire suite à une augmentation future du volume intravasculaire, comprenant les étapes de :
a) mesure in vitro du taux d'un peptide natriurétique du groupe de l'ANP ou du NT-proANP et/ou du BNP ou du NT-proBNP ;
b) diagnostic du risque auquel est exposé le patient en comparant le taux mesuré à au moins un taux connu associé à différents degrés de risque chez un patient.

2. Procédé selon la revendication 1, dans lequel le peptide natriurétique est le NT-proBNP.

3. Procédé selon la revendication 1 ou 2, dans lequel un taux plasmatique supérieur à 60 et inférieur à 1000 pg/ml de NT-proBNP chez un patient de sexe masculin est associé à une augmentation du risque de souffrir d'une complication cardio-vasculaire, ladite augmentation du risque indiquant qu'un traitement peut être adapté.

4. Procédé selon la revendication 1 ou 2, dans lequel un taux plasmatique supérieur à 120 et inférieur à 1000 pg/ml de NT-proBNP chez une patiente de sexe féminin est associé à une augmentation du risque de souffrir d'une complication cardio-vasculaire, ladite augmentation du risque indiquant qu'un traitement peut être adapté.

5. Procédé selon la revendication 1 ou 2, dans lequel un taux plasmatique de 1000 à 5000 pg/ml de NT-proBNP est associé à une forte augmentation du risque de souffrir d'une complication cardio-vasculaire, ladite forte augmentation du risque indiquant que l'on peut reconsidérer le fait de savoir si une quelconque augmentation du volume intravasculaire peut être tolérée.

6. Procédé selon la revendication 1 ou 2, dans lequel un taux plasmatique supérieur à 5000 pg/ml de NT-proBNP est associé à une très forte augmentation du risque de souffrir d'une complication cardio-vasculaire, ladite très forte augmentation du risque indiquant que l'on peut reconsidérer le fait de savoir si une quelconque augmentation du volume intravasculaire peut être tolérée et dans lequel une hospitalisation immédiate et/ou un traitement cardiaque intensif peut également être envisagé.

7. Procédé selon l'une quelconque des revendications 1 à 6, dans lequel l'augmentation du volume intravasculaire est provoquée par une maladie, en particulier par une sepsie ou une gammapathie.

8. Procédé selon l'une quelconque des revendications 1 à 7, dans lequel l'augmentation du volume intravasculaire est due à une cause artificielle, via un liquide à administrer par perfusion ou par transfusion, ou via un médicament à administrer.

9. Procédé selon la revendication 8, dans lequel le médicament à administrer est choisi parmi le groupe constitué par :
a) des antirhumatoïdes non stéroïdiens ;
b) des corticostéroïdes ;
c) des antidiabétiques ;
d) des oestrogènes ;
e) des inhibiteurs du TNF ;
f) des inhibiteurs sélectifs de la cyclo-oxygénase-2.

10. Procédé selon l'une quelconque des revendications 1 à 9, dans lequel la complication cardio-vasculaire est une maladie coronarienne, un angor stable, un syndrome coronarien aigu, un angor instable, un infarctus du myocarde, un dysfonctionnement du ventricule gauche, une insuffisance cardiaque congestive ou une congestion pulmonaire.

11. Procédé selon la revendication 10, dans lequel la complication cardio-vasculaire est une congestion pulmonaire.

12. Procédé selon l'une quelconque des revendications 1 à 11, dans lequel le taux de l'hormone cardiaque est mesuré en utilisant un ligand à liaison spécifique, une matrice, un dispositif microfluidique, un analyseur à chimioluminescence ou un dispositif robotique.

13. Procédé selon la revendication 12, dans lequel le ligand à liaison spécifique est un anticorps ou un aptamère.

14. Procédé selon l'une quelconque des revendications 1 à 13, dans lequel le ligand à liaison spécifique est marqué.

15. Utilisation d'un moyen diagnostique capable de mesurer in vitro le taux chez un patient d'un peptide natriurétique du groupe de l'ANP, du NT-proANP et/ou du BNP ou du NT-proBNP afin de diagnostiquer le risque auquel est exposé le patient, qui ne présente aucun symptôme d'une maladie cardio-vasculaire conformément à la classification NYHA et qui n'a pas d'histoire de complications cardio-vasculaires, de souffrir d'une complication cardio-vasculaire suite à une augmentation future du volume intravasculaire.

16. Procédé de prise de décision concernant l'administration future à un patient, qui ne présente aucun symptôme d'une maladie cardio-vasculaire conformément à la classification NYHA et qui n'a pas d'histoire de complications cardio-vasculaires, une perfusion, une transfusion ou un médicament provoquant une surcharge en volume, comprenant :
a) la mesure in vitro du taux d'un peptide natriurétique du groupe de l'ANP, du NT-proANP et/ou du BNP, du NT-proBNP chez le patient ;
b) la comparaison du taux mesuré à au moins un taux connu associé à différents degrés de risque chez un patient ;
c) de manière facultative, faire examiner le patient par un cardiologue ;
d) recommander ou réfréner l'administration de la perfusion, de la transfusion ou du médicament, de manière facultative en prenant en compte le résultat de l'examen du patient par le cardiologue.

17. Procédé selon la revendication 16, dans lequel le procédé est destiné à prendre une décision concernant l'administration future à un patient, qui ne présente aucun symptôme de complications cardio-vasculaires, d'un médicament donnant lieu à une surcharge en volume et dans lequel le médicament est un inhibiteur sélectif de la cyclo-oxygénase-2.
